# FASCICULE DE BREVET EUROPEEN

(11) **EP 3 188 798 B1**
(45) Date de publication et mention de la délivrance du brevet: **15.03.2023**
(21) Numéro de dépôt: 15759732.9
(22) Date de dépôt: 01.09.2015
(51) Int. Cl.: A61N 7/02, A61B 18/12, A61B 18/18, A61B 18/20, A61B 5/01, A61B 5/055

(54) **SYSTÈME POUR L'ABLATION OU LE CONTRÔLE D'UNE ZONE DU COEUR PAR ULTRASONS**
SYSTEM ZUR ABLATION EINER HERZREGION MITTELS ULTRASCHALL
SYSTEM FOR ULTRASOUND ABLATION OF AN AREA OF THE HEART

(30) Priorité: 02.09.2014 FR 1458206
(43) Date de publication de la demande: 12.07.2017
(73) Titulaire: Université de Bordeaux, 33000 Bordeaux (FR); Institut National de la Santé et de la Recherche Médicale (INSERM), 75013 Paris (FR); Centre Hospitalier Universitaire de Bordeaux, 33404 Talence Cedex (FR)
(72) Inventeur: MARQUET, Fabrice, F-33300 Bordeaux (FR); BOUR, Pierre, F-33400 Talence (FR); QUESSON, Bruno, F-33000 Bordeaux (FR); VAILLANT, Fanny, F-33000 Bordeaux (FR)
(74) Mandataire: Cabinet Camus Lebkiri
(86) Numéro de dépôt international: PCT/EP2015/069968
(87) Numéro de publication internationale: WO 2016/034594

(56) Documents cités:
- EP-A1- 2 312 303
- EP-A2- 0 627 206
- US-A1- 2013 150 756
- US-A1- 2013 150 756
- US-A1- 2013 184 697

## Description

### DOMAINE

Le domaine de l'invention concerne les méthodes de contrôle d'un faisceau de signaux ultrasonores focalisés pour établir une calibration dudit faisceau et pour établir un contrôle actif lors d'une opération d'ablation de diverses régions du cœur. En outre, le domaine de l'invention concerne les méthodes d'ablation à partir de signaux ultrasonores. Par ailleurs, le domaine de l'invention se rapporte aux systèmes permettant la mise en oeuvre des procédés de l'invention. Les procédés en tant que tels ne font pas partie de l'invention revendiquée.

### ETAT DE L'ART

Aujourd'hui, il existe différentes solutions permettant de traiter les troubles du rythme cardiaque appelés « arythmies cardiaques ». Une solution pour traiter certaines zones causant des dysfonctionnements cardiaques et de procéder à une ablation de ces dernières.

L'ablation d'une zone d'un organe peut être effectuée par un cathéter intracardiaque introduit dans l'organe. Dans ce cas de figure, l'opération est suivie à partir d'un système d'imagerie pour guider l'opération d'ablation. Lorsque cette opération est conduite dans le coeur, un inconvénient est qu'elle nécessite de grandes précautions pour maintenir l'environnement d'intervention stérile. En outre, cette méthode nécessite différents cathéters à usage unique, elle présente donc un coût important à chaque intervention. En outre, cette dernière opération comporte des risques et le contrôle de la zone à ablater est difficile à mettre en oeuvre.

Une autre solution plus récente est l'ablation d'une zone d'un organe par un moyen extracorporel. Une solution consiste, par exemple, à utiliser des signaux à une certaine puissance et interférant pour créer une zone d'échauffement afin de brûler des cellules ou des tissus de l'organe.

Cette solution peut être calibrée lorsque l'organe est relativement stable. En revanche, cette solution est beaucoup plus difficile à mettre en oeuvre et à guider lorsque l'opération se déroule sur un organe en mouvement. L'opération devient particulièrement sensible et potentiellement dangereuse si un contrôle n'est pas effectué. C'est le cas notamment d'une ablation sur une zone du cœur. En effet, le coeur étant en mouvement, il est difficile de détruire des tissus cardiaques défaillants avec la certitude ne pas endommager les tissus voisins non défaillants. En particulier, l'utilisation d'un générateur d'ondes ultrasonores nécessite de focaliser un faisceau dans une région du coeur tout en évitant les os des côtes. Aujourd'hui, les solutions évoquées dans l'art antérieur ne permettent d'effectuer une ablation sans causer des dommages sur la paroi transcostale.

L'utilisation de signaux ultrasonores pour l'ablation de certaines zones du coeur causant un dysfonctionnement est donc, à ce stade, difficilement maîtrisée. En effet, le contrôle du signal ultrasonore généré est indispensable pour déterminer et traiter avec précision la région à traiter et ainsi éviter tout dommage indésirable sur des régions limitrophes à la zone à ablater.

A cet effet, le brevet US n° 2006/005265 traite de la stimulation cardiaque par ultrasons contrôlée par le couplage d'un électrocardiographe mesurant l'activité électrique du coeur avec un système d'imagerie. Cette demande de brevet évoque notamment la possibilité de réaliser une ablation au moyen d'un faisceau d'ultrasons en chauffant localement les tissus pour les détruire.

En revanche, cette solution reste muette sur les contrôles nécessaires à la mise en oeuvre d'un tel procédé d'ablation pour éviter de causer tout dommage thermique ou mécanique au-delà de la zone concernée par l'ablation.

Une autre solution exposée dans la demande de brevet US2013/0184697 permet de réaliser une ablation d'une zone du coeur au moyen de signaux ultrasonores. La demande décrit en particulier comment détecter des zones électriquement défaillantes provoquant des arythmies cardiaques à partir d'une matrice d'électrodes agencées sur le patient. Une cartographie électrique d'une région comprenant le coeur est réalisée afin de détecter les zones électriquement défaillantes. Cette solution impose la résolution d'un problème inverse de mesures d'activités électriques sur un ensemble d'électrodes afin de reconstituer la cartographie électrique du coeur.

Cette méthode nécessite donc une longue calibration des électrodes sur un patient et un coût de calculs important pour détecter les zones provoquant des arythmies cardiaques.

Cette méthode indique qu'un contrôle de la température peut être effectué mais elle ne permet pas un contrôle en temps réel de certains paramètres avant ou pendant l'ablation permettant de sécuriser l'opération d'ablation et de limiter les dommages pouvant être causés sur une zone connexe à la zone concernée par l'ablation.

Le document EP 0 627 206 intitulé « Method and apparatus for ultrasound medical treatment », le document US 2013/150756 intitulé « Rib identification for transcostal focused ultrasound surgery » et le document EP 2 312 303 intitulé « Magnetic résonance imaging system and method for detecting a gas bubble » sont connus de l'état de la technique.

### RESUME DE L'INVENTION

L'invention vise à pallier les inconvénients précités.

L'invention vise à proposer une méthode de contrôle de paramètres permettant de calibrer et guider l'opération d'ablation en toute sécurité. En outre, l'invention concerne un procédé d'ablation comprenant un contrôle actif et dynamique de la zone d'ablation de manière à vérifier la conformité de l'opération avec la prévision attendue. En outre, le procédé d'ablation de l'invention permet d'ajuster dynamiquement des caractéristiques d'un faisceau, puissance, déflexion et durée, automatiquement en fonction d'un paramètre contrôlé avant ou pendant l'opération d'ablation.

Les procédés ou méthodes en tant que tels ne font pas partie de l'invention revendiquée.

Divulguée est une méthode de contrôle d'une zone du coeur.
La méthode comprend :
▪ une acquisition du rythme de l'électrocardiogramme du coeur ;
▪ une acquisition d'au moins une image d'une région du coeur dans laquelle une zone ciblée est repérée, ladite au moins une image étant acquise de manière synchrone avec le rythme de l'électrocardiogramme du coeur par un système d'imagerie ;
▪ une génération d'un premier faisceau de signaux ultrasonores focalisé dans la zone ciblée lesdits faisceaux étant émis par un réseau de phase, lesdits signaux étant configurés en phase pour générer au moins une impulsion dans une zone focalisée, ladite au moins une impulsion étant synchronisée sur le rythme de l'électrocardiogramme du coeur, l'impulsion ayant une amplitude et une durée prédéfinies ;
▪ un asservissement dynamique de la position de la zone focalisée sur la position de la zone ciblée au moyen d'un système de positionnement permettant de mesurer des mouvements respiratoires du coeur dans un référentiel lié au réseau de phase et d'en déduire un paramètre de compensation pour calculer une nouvelle position de la zone ciblée, ledit réseau de phase appliquant automatiquement un paramètre de phase à chaque signal pour défléchir le faisceau vers la nouvelle position de la zone ciblée ;
▪ une détermination d'une température au niveau de la zone focalisée à partir d'une acquisition d'au moins une image de ladite zone au moyen d'un système d'imagerie.

Un avantage d'une telle méthode est d'effectuer un test sur la zone ciblée Z_{C} afin de calibrer le faisceau de signaux ultrasonores en générant l'amplitude et la durée adéquates lors de l'ablation d'une zone du coeur.

Un avantage d'une telle méthode est d'effectuer un contrôle de certains paramètres tels que la température, la déformation tissulaire ou l'activité électrique sur la zone ciblée afin de calibrer le faisceau de signaux ultrasonores en générant l'amplitude et la durée adéquates lors de l'opération d'ablation.

Divulguée est en outre :
▪ une détermination d'une déformation tissulaire dans la zone focalisée en réponse à au moins une impulsion du premier faisceau focalisé.

Divulguée est en outre:
▪ Une détermination par un système d'imagerie de l'image de la paroi transcostale projetée dans un plan image du réseau de phase en prenant en considération la position et l'orientation du réseau de phase ;
▪ Une désactivation des éléments du réseau de phase en fonction de la position desdits éléments vis-à-vis de la position de l'image projetée de la paroi transcostale.

Selon une divulgation, la méthode comprend
▪ Un asservissement dynamique de la désactivation et de l'activation des éléments du réseau de phase en fonction du calcul de chaque paramètre de phase appliqué à chacun des signaux.

Selon une divulgation, la température dans la zone focalisée et la déformation tissulaire sont déterminées par un même système d'imagerie, ledit système d'imagerie étant un système d'imagerie IRM, les données acquises par le système d'imagerie IRM permettant de déduire une déformation locale du tissu induite par la pression ultrasonore générée par le faisceau de signaux ultrasons et une élévation de température locale induite par l'énergie générée localement par le faisceau de signaux ultrasons.

Selon une divulgation, une comparaison de la position de la zone focalisée déterminée par un système d'imagerie et de la position de la zone ciblée déterminée par le système de positionnement génère au moins une donnée pour calibrer des éléments du réseau de phase de manière à faire correspondre une position de la zone focalisée avec la position de la zone ciblée.

Selon une divulgation, le système de positionnement peut être notamment :
▪ un système d'imagerie IRM, les positions étant calculées à partir d'un traitement d'images ;
▪ un système de positionnement comprenant au moins un émetteur émettant des ondes ultrasonores et une pluralité de capteurs à ultrasons détectant les ondes réfléchies, les positions étant déterminées par triangulation.

Selon une divulgation, la méthode de contrôle comprend une calibration d'un signal généré dans la zone focalisée par la définition de paramètres comprenant au moins un niveau de l'amplitude et une durée d'une d'impulsion en fonction d'au moins une donnée parmi lesquelles :
▪ d'une consigne de température dans la zone focalisée et/ou et sur les zones voisines et/ou sur les côtes de la paroi transcostale ;
▪ d'une consigne de la déformation tissulaire dans la zone focalisée et/ou ;
▪ d'une consigne d'un niveau de cavitation dans la zone focalisée et/ou ;
▪ d'une détection d'un mouvement de la zone focalisée dans un repère lié au réseau de phase et/ou ;
▪ d'une consigne d'activité électrique dans la zone focalisée (Z_{F}).

Selon une divulgation, les étapes de la méthode de contrôle sont effectuées dans différentes zones ciblées (Z_{c}) du coeur, la méthode comprenant en outre après l'application d'un faisceau de signaux focalisés (F_{US}) :
▪ Une vérification de différentes valeurs représentant soit des déformations tissulaires de chaque zone ciblée (Z_{c}), soit des niveaux électriques mesurés à proximité de ou dans chaque zone ciblée (Z_{c}), lesdites valeurs mesurées étant comparées à des seuils donnés.

Un autre objet de la divulgation concerne une méthode d'ablation d'une zone donnée d'un coeur par génération d'un faisceau de signaux ultrasonores focalisé, caractérisée en ce qu'elle comprend :
▪ une acquisition du rythme de l'électrocardiogramme du coeur ;
▪ une détermination d'au moins une position d'une zone ciblée dans le coeur ;
▪ une génération d'un faisceau de signaux ultrasonores focalisé dans la zone ciblée dont :
   ▪ une amplitude est continue et est comprise dans la gamme de valeurs [1-100 MPa] ;
   ▪ une durée d'application du faisceau focalisé étant appliquée sur une période supérieure à 1ms ;
▪ un asservissement de la position de la zone focalisée du faisceau sur la position calculée en temps réel de la zone ciblée au moyen d'un système de positionnement ;
▪ un contrôle actif en temps réel d'une température dans la zone focalisée ou d'une zone voisine à la zone focalisée à partir d'une acquisition d'image(s).

Selon une divulgation, la durée d'application du faisceau focalisé comprend au moins une microcoupure d'une durée supérieure à 1 ms pendant laquelle l'étape du contrôle actif comprend au moins une mesure de l'élasticité du tissu de la zone ciblée.

Selon une divulgation, contrôle actif comprend une mesure d'un niveau de cavitation de la zone focalisée au moyen d'un capteur à ultrasons détectant un spectre de fréquences ultrasonores dans ou à proximité de la zone focalisée, le niveau de cavitation étant déduit d'un niveau de bruit spectral.

Selon une divulgation, le contrôle actif comprend une mesure de l'élasticité du tissu déduite d'une mesure d'un temps de relaxation du tissu :
▪ soit à partir d'une image d'un système d'imagerie IRM
▪ soit à partir d'une image d'un système d'imagerie ultrasonore par une mesure d'élastométrie.

Selon une divulgation, la mesure de l'élasticité du tissu de la zone ciblée et/ou la mesure du niveau de cavitation détermine automatiquement une donnée correspondant à la durée à appliquer du faisceau focalisé après la microcoupure et/ou détermine l'instant auquel sera générée une prochaine microcoupure.

Selon une divulgation, un asservissement dynamique de la position de la zone focalisée sur la position de la zone ciblée au moyen d'un système de positionnement permet de mesurer des mouvements respiratoires du coeur dans un référentiel lié au réseau de phase et d'en déduire un premier paramètre de compensation pour calculer une nouvelle position de la zone ciblée, ledit réseau de phase appliquant automatiquement un paramètre de phase à chaque signal pour défléchir le faisceau vers la nouvelle position de la zone focalisée.

Selon une divulgation, un asservissement dynamique de la position de la zone focalisée sur la position de la zone ciblée au moyen d'un système de positionnement permet de mesurer des mouvements de contractions du coeur lors de la survenance du complexe QRS dans un référentiel lié au réseau de phase et d'en déduire un second paramètre de compensation pour calculer une nouvelle position de la zone ciblée, ledit réseau de phase appliquant automatiquement un paramètre de phase à chaque signal pour défléchir le faisceau vers la nouvelle position de la zone focalisée.

Selon une divulgation, le système de positionnement permet de prédire les moments de la survenance du complexe QRS correspondant à la contraction du coeur et de générer :
▪ une consigne d'arrêt de l'émission d'au moins un faisceau pendant la durée de la contraction et :
   ▪ une consigne de réactivation du faisceau asservi après la fin de la contraction.

Selon un mode de réalisation, la méthode d'ablation comprend :
▪ Une détermination par un système d'imagerie d'une image d'une paroi transcostale projetée dans un plan image du réseau de phase en prenant en considération la position et l'orientation du réseau de phase ;
▪ Une désactivation des éléments du réseau de phase lorsque les signaux émis desdits éléments sont susceptibles d'intercepter l'image projeté de la paroi transcostale.

Selon une divulgation, le contrôle actif comprend :
▪ Un asservissement dynamique de la désactivation et de l'activation des éléments du réseau de phase en fonction de la position de l'image projeté de la paroi transcostale sur les éléments du réseau de phase.

Selon une divulgation, un second contrôle actif de zones voisines à la zone focalisée est réalisé, ledit second contrôle actif comportant la mesure des paramètres suivants en temps réel :
▪ une température d'au moins une zone voisine (Z_{V}) de la zone focalisée et ;
▪ une déformation tissulaire dans au moins une zone voisine (Z_{V}) de la zone focalisée en réponse à au moins une impulsion du premier faisceau focalisé et/ou ;
▪ un niveau de cavitation dans au moins une zone voisine (Z_{V}) de la zone focalisée en réponse à au moins une impulsion du premier faisceau focalisé.

Selon une divulgation, la méthode d'ablation comprend préalablement :
▪ une calibration d'un faisceau focalisé après l'exécution de la méthode de contrôle ;
▪ ladite calibration permettant de déterminer les paramètres suivants :
   ▪ une amplitude configurée de sorte que la pression acoustique appliquée dans la zone focalisée est continue et est comprise dans la gamme de valeurs [1-100 MPa] ;
   ▪ une durée d'application du faisceau focalisé étant appliquée sur une période supérieure à 1ms pouvant comporter des microcoupures.

Selon une divulgation, une méthode d'ablation d'une pluralité de zones ciblées d'un coeur par génération d'un faisceau de signaux ultrasonores focalisé en une pluralité de points comprend préalablement :
▪ une calibration multipoints d'un faisceau focalisé après l'exécution de la méthode de contrôle;
▪ une configuration d'un réseau d'éléments pour générer une pluralité de faisceaux dans une pluralité de zones focalisées.

Selon une divulgation, la méthode de contrôle est appliquée successivement à la méthode d'ablation pour vérifier qu'une déformation tissulaire ou une activité électrique dans cette zone est inférieure à un seuil prédéterminé, ladite zone ciblée du coeur étant alors indiquée comme « non répondante ».

L'objet de l'invention concerne un système selon la revendication 1.

Avantageusement, le système pour l'ablation ou le contrôle d'une zone du coeur par ultrasons est capable de mettre en oeuvre les étapes des méthodes de contrôle et d'ablation.

### BREVE DESCRIPTION DES FIGURES

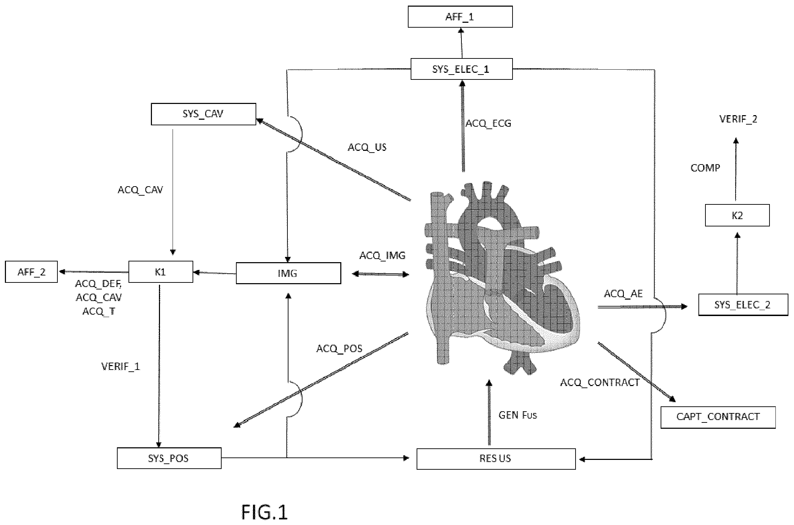

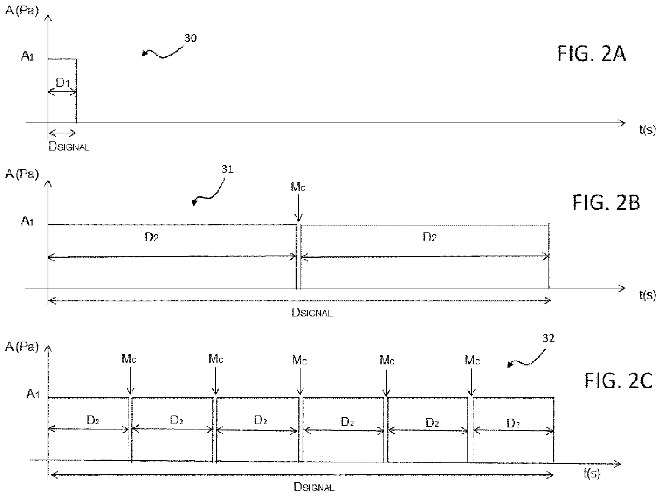

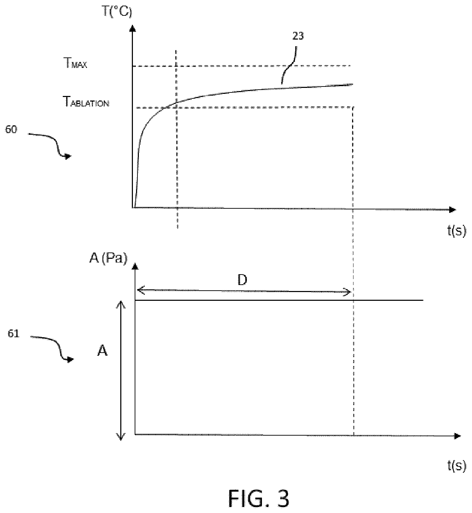

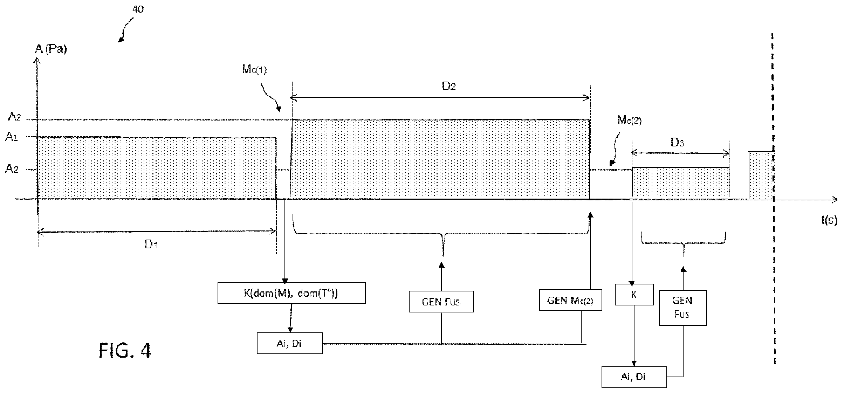

D'autres caractéristiques et avantages de l'invention ressortiront à la lecture de la description détaillée qui suit, en référence aux figures annexées, qui illustrent :
▪ figure 1: un schéma général de fonctionnement du procédé de l'invention;
▪ figure 2A : un diagramme représentant un exemple de stimulation d'une zone ciblée du coeur pour calibrer un faisceau pour une application d'ablation ;
▪ figure 2B : un diagramme représentant un premier exemple de faisceau pour l'ablation d'une zone ciblée du coeur ;
▪ figure 2C : un diagramme représentant un second exemple de faisceau pour l'ablation d'une zone ciblée du coeur;
▪ figure 3 : les diagrammes représentant l'évolution de la température de la zone ciblée en fonction d'une amplitude et une durée d'application d'un faisceau de signaux ultrasonores;
▪ figure 4 : un diagramme représentant un troisième exemple de faisceau pour l'ablation d'une zone ciblée du coeur.

### DESCRIPTION

### Définitions et introduction du principe

La figure 1 représente un schéma général de fonctionnement d'un mode de réalisation du procédé divulguée. Le système de la figure 1 représente différents équipements qui sont décrits successivement.

On nommera dans la suite de la description indifféremment une « amplitude » et une « pression acoustique ».

Une « impulsion » peut s'interpréter au niveau du signal comme une émission pendant une certaine durée mais aussi comme le phénomène induit localement par ce signal. Le phénomène induit correspond à une poussée ultrasonore locale résultant d'une force de radiation ultrasonore. Cette dernière poussée est susceptible d'exercer directement une poussée mécanique sur les tissus cardiaques.

On appelle « faisceau focalisé » : un ensemble de signaux provenant de différents éléments d'un réseau de phase dont les caractéristiques en fréquence et en phase permettent de générer des interférences constructives ou cohérentes en une zone focalisée.

Le réseau de phase permet, selon les modes de réalisations, de définir une ou plusieurs zones focalisées dans le coeur grâce à une configuration particulière des phases de chaque élément du réseau de phase. Les différentes zones focalisées sont obtenues en faisant interférer les signaux de différents éléments du réseau de phase en différents points.

Le procédé divulgué permet d'effectuer des mesures de paramètres de contrôle, tels que la déformation tissulaire ACQ_DEF, la température ACQ_T, l'activité électrique ACQ_AE ou encore la présence d'une contraction cardiaque ACQ_CONTRACT, pendant ou conséquemment à l'application du faisceau focalisé. En outre, les procédés de l'invention permettent de mesurer un niveau représentatif du phénomène de cavitation ACQ_CAV dans ou à proximité de la zone focalisée ainsi qu'une mesure de position ou de déplacement ACQ_POS de la zone ciblée Z_{C}. La mesure des déplacements de la zone ciblée Z_{C} permettent par exemple de prendre en compte les mouvements respiratoires ou les contractions du coeur dans l'asservissement du faisceau focalisé.

On parle indifféremment de « déformation tissulaire » et de « déplacement des tissus » dans la description pour décrire l'effet causé par la génération d'une poussée ultrasonore sur les tissus cardiaques.

Un réseau de phase RES_US permet de générer un ou plusieurs faisceaux focalisés dans une ou plusieurs zones focalisées. Chaque zone focalisée est asservie en position par un système de positionnement SYS_POS, par exemple assuré au moyen d'un système d'imagerie IMG ou une sonde intracardiaque ou éventuellement par les deux.

L'asservissement de la ou des position(s) du ou des faisceaux focalisés en un ou plusieurs point(s), appelé(s) « zone focalisée », est réalisée au moyen d'une consigne de position, appelée « zone ciblée ».

Le mécanisme d'asservissement du faisceau focalisé sur une zone ciblée est avantageusement réalisé de manière synchrone entre le système de positionnement et le réseau de phase grâce à l'acquisition de l'ECG qui constitue une référence temporelle commune aux différents équipements.

Une particularité du procédé de l'invention permet de prendre en compte un paramètre de déplacement de la consigne de position de la zone ciblée causé par les mouvements respiratoires et/ou la contraction du coeur pour asservir convenablement le réseau de phase. Une nouvelle position ciblée est alors calculée au moyen d'un calculateur couplé au système de positionnement pour adapter la consigne de position au réseau de phase. Selon le procédé de l'invention, le réseau de phase est capable de générer un paramètre de phase à chaque signal émis par un élément du réseau pour défléchir le faisceau et asservir le point focalisé Z_{F} sur la nouvelle position de la zone ciblée Z_{C}.

Par ailleurs, outre les phénomènes respiratoires, il subsiste des mouvements de contractions du coeur, particulièrement pendant le complexe QRS de l'ECG. De manière à s'affranchir des mouvements de contraction du coeur dans l'asservissement de la zone focalisée sur la zone ciblée, le faisceau focalisé peut :
▪ soit être interrompu lors de l'apparition du complexe QRS visible sur l'ECG ;
▪ soit être complété par l'application d'une seconde correction de déflexion pour compenser le déplacement du coeur lors de l'apparition du complexe QRS.

L'invention permet de mettre en oeuvre les deux solutions lors du procédé de contrôle ou lors du procédé d'ablation.

### Acquisition ECG

Une première étape des procédés de l'invention, notée ACQ_ECG, comprend l'acquisition d'un électrocardiogramme, noté ECG, d'un patient ou d'un animal à partir d'un premier système électrique noté SYS_ELEC_1. Selon un mode de réalisation, l'électrocardiogramme permet d'acquérir le rythme des battements d'un coeur qui s'affiche sur un afficheur, noté AFF_1.

Selon un mode de réalisation, l'acquisition de l'ECG est réalisée en plaçant des électrodes à la surface du corps d'un patient ou d'un animal. Cette solution permet de mesurer l'activité électrique d'un coeur de manière non invasive. Selon une variante de réalisation, l'ECG peut être acquis au moyen de douze ou seize dérivations selon des modalités connues d'agencement et de positionnement d'électrodes.

Selon un mode de réalisation, un cathéter spécifique est positionné dans une cavité du coeur pour la mesure d'une activité électrique locale. Dans ce cas, le procédé de l'invention permet également de comparer les activités électriques acquises par le cathéter électrique et le dispositif permettant d'obtenir l'ECG. Ce moyen est noté SYS_ELEC_2 sur la figure 1.

### - Synchro applications

Outre son affichage, l'ECG est utilisé selon le procédé de l'invention pour synchroniser différents équipements ensemble. La synchronisation des différents équipements permet de générer des actions de manière synchrone dans une ou plusieurs régions du coeur.

Les actions synchronisées comprennent notamment :
▪ la balistique : c'est-à-dire l'asservissement de la position de la zone focalisée sur la position de la zone ciblée ou en prenant en compte par exemple la fréquence de contraction du coeur dans l'asservissement de la balistique ou l'arrêt de l'émission des faisceaux. Cette synchronisation prend en compte les mouvements du coeur ainsi qu'au besoin l'évitement de la paroi transcostale et/ou ;
▪ le contrôle : c'est-à-dire la mesure de certains paramètres de contrôle pendant ou conséquemment à la génération du faisceau.

### - Synchronisation du faisceau

La génération du faisceau est avantageusement synchronisée pendant une dépolarisation des tissus cardiaques. Pour le ventricule, le faisceau focalisé est donc généré préférentiellement pendant la période QT de l'ECG. Avantageusement, cette synchronisation limite la génération d'une stimulation cardiaque pouvant provoquer une dépolarisation des tissus cardiaques et éventuellement une contraction du coeur pendant lors d'une opération d'ablation.

### - Synchronisation de l'imagerie

En ce qui concerne l'acquisition d'au moins une image ACQ IM, elle est préférentiellement synchronisée avec l'ECG également. C'est-à-dire que l'image est acquise à des instants donnés de l'ECG. Les instants d'acquisition d'image vis-à-vis de l'ECG peuvent être configurés par un opérateur ou peuvent être automatiquement déduits en fonction d'une consigne de synchronisation.

Dans un mode de réalisation, la génération GEN F_{US} d'un faisceau de signaux ultrasonores focalisé dans une zone ciblée du coeur et l'acquisition d'images ACQ IMG sont synchronisées ensemble avec l'ECG. On entend par « synchronisé » du point de vue du réseau de phase, le fait que la génération du faisceau ultrasonore GEN F_{US} est déclenchée à un instant déterminé de l'ECG. Cette synchronisation permet de provoquer un effet physiologique souhaité dans une zone du coeur en prenant en considération l'état de polarisation du tissu cardiaque.

### Acquisition d'images

Une seconde étape, notée ACQ IMG, comprend l'acquisition d'au moins une image d'une région du coeur, par un système d'imagerie noté IMG, dans laquelle une zone ciblée Z_{C} est repérée, notée ACQ_Zc.

Selon un mode de réalisation, l'acquisition de l'image est réalisée au moyen d'un système d'imagerie IRM. Basé sur les propriétés magnétiques des atomes, le système d'imagerie IRM consiste à appliquer un champ magnétique aux noyaux atomiques puis à stimuler par radiofréquences lesdits noyaux atomiques. La reconstitution d'une image est alors possible à partir du signal émis lors de phase de relaxation et recueilli par des capteurs électromagnétiques.

Avantageusement, le système d'imagerie IRM couplé avec un calculateur K₁ permet de :
▪ définir une position d'une zone ciblée Z_{C} comme une consigne d'asservissement du réseau de phase de signaux focalisés ; la position de la zone ciblée Zc peut également être définie par un autre équipement de positionnement ;
▪ déduire une déformation tissulaire et/ou une variation de déformation tissulaire à proximité de ou dans la zone focalisée, l'IRM dans ce mode est appelée plus communément IRM-ARFI ; l'IRM ARFI permet de mesurer la dureté ou l'élasticité des tissus suite à une augmentation de la température dans une zone identifiée ;
▪ déduire une température et/ou une variation de températures à proximité de ou dans la zone focalisée, l'IRM dans ce mode est appelée plus communément IRM-T ;
▪ déterminer la projection de la paroi transcostale d'un patient dans le plan image du réseau de phase pour activer ou désactiver les éléments du réseau de phase susceptibles de causer des dommages à la paroi osseuse en prenant en considération un paramètre de déflexion du faisceau.

Selon un autre mode de réalisation, l'invention comprend une acquisition d'une image au moyen d'un système d'imagerie ultrasonore. Ce système peut être utilisé en complément d'un système d'imagerie IRM ou en substitution de celui-ci pour les mesures de déplacements du tissu. Un système d'imagerie ultrasonore, appelé échocardiographie, est basé sur l'émission d'ondes acoustiques dans l'organisme. Lesdites ondes sont réfléchies différemment selon le type de structures anatomiques rencontrées. Le signal recueilli, correspondant aux échos des ondes émises, permet de reconstituer une image d'une partie de l'anatomie d'un patient ou d'un animal.

Un système d'imagerie échographique peut également être utilisé en substitution d'un système IRM pour mesurer la température dans la zone focalisée et/ou dans une zone voisine à la zone focalisée.

Selon un autre mode de réalisation, l'acquisition d'une image est réalisée au moyen d'un système d'imagerie à rayons X. Ce système d'imagerie est basé sur l'émission de rayons X sur les tissus. La mesure de l'atténuation des rayons X par les tissus permet de reconstituer des images en 2D ou 3D des structures anatomiques comme par exemple le coeur. Ce système peut être utilisé en complément d'un système d'imagerie IRM ou en substitution de celui-ci pour les mesures de balistique, c'est-à-dire de la position de la zone focalisée. La zone ciblée Z_{C} peut être éventuellement définie à partir du système d'imagerie.

Selon un autre mode de réalisation, l'acquisition d'une image est réalisée au moyen d'un système d'imagerie, appelé tomoscintigraphie, par émission de positons (TEP). Ce système d'imagerie est basé sur la détection de radiations gamma émises par une substance radioactive injectée en faible quantité dans l'organisme, ce qui permet d'acquérir des images en coupe de certains organes comme par exemple le coeur. Ce système peut être utilisé en complément d'un système d'imagerie IRM ou en substitution de celui-ci pour les mesures de balistique, c'est-à-dire de la position de la zone focalisée et/ou de la zone ciblée. La zone ciblée Z_{C} peut être éventuellement définie à partir du système d'imagerie.

Les systèmes d'imagerie peuvent être, au besoin, couplés à des agents de contraste injectés ou ingérés de manière à améliorer la visualisation des organes explorés.

### Système de positionnement

Un système de positionnement est utilisé lors de l'exécution des procédés de l'invention pour réaliser différents fonctions :
▪ une première fonction est de définir la position d'une zone ciblée que l'on souhaite atteindre. A cet effet, selon un exemple, le système de positionnement peut être couplé à un système d'imagerie pour récupérer sur l'image la position ACQ_IMG d'une zone ciblée identifiée.
▪ une seconde fonction est de calibrer la balistique de chaque dispositif ou système réalisant une fonction des procédés de l'invention. Ainsi, les systèmes d'imagerie et le réseau de phase peuvent être calibrés en position avec un système de positionnement.
▪ Une troisième fonction est d'asservir la position de la zone focalisée Z_{F} sur la position de la zone ciblée Z_{C}. Pour cela une consigne est générée vers le réseau de phase pour que le faisceau soit défléchi correctement.
▪ Une quatrième fonction est de générer une consigne d'activation ou de désactivation des éléments du réseau de phase en fonction de la position projetée de la paroi transcostale dans le plan du réseau de phase. Cette fonction peut être assurée directement par le système d'imagerie vers le réseau de phase. L'utilisation du système de positionnement peut permettre d'améliorer la vitesse d'asservissement sans à avoir à être limité par les temps d'acquisition de l'image. Dans ce dernier cas, les calculs des positions de l'image projetée de la paroi transcostale peuvent être mémorisés dans le système de positionnement et les changements de positions du patient peuvent être calculés à partir d'une position de référence.

Selon un mode de réalisation, le système de positionnement comprend des capteurs ultrasonores extracorporels et un émetteur extracorporel dont les émissions de signaux ultrasonores ACQ_US sont réfléchies et détectées par les capteurs. Une position donnée du coeur peut être obtenue par triangulation. Quatre capteurs des signaux permettent d'obtenir une bonne précision des positions de zones dans le coeur. Un avantage de cette solution est que l'asservissement d'une position de l'espace peut être plus rapide que par l'utilisation d'un système d'imagerie. En effet, cette solution nécessite moins de données à acquérir et à traiter. Les récepteurs acquièrent les variations sur une ligne de l'espace 3D.

Selon une variante de réalisation, l'émetteur est un émetteur dédié au système de positionnement. Selon une autre variante, l'émetteur peut être par exemple un élément du réseau de phase notamment pour établir la calibration de position de départ.

Les récepteurs du réseau de phase peuvent être utilisés. On préféra pour améliorer les précisions des mesures des capteurs d'ondes ultrasonores dédiés au système de positionnement.

Dans un autre mode de réalisation, le système de positionnement est réalisé au moyen de capteurs positionnés sur la peau.

Lorsque la position de la zone focalisée Z_{F} ou de la zone ciblée Z_{C} est déterminée à partir d'un système d'imagerie IMG, selon un exemple de réalisation, elle peut être reconnue automatiquement à partir d'un traitement d'images. A cet effet, un indicateur basé sur des variations de paramètres relatifs aux pixels de l'image peut être généré pour reconnaître automatiquement une zone spécifique du coeur. Selon un autre exemple de réalisation, la zone ciblée Z_{C} peut être identifiée au moyen d'un outil de traitement d'images avec l'usage d'une souris ou d'un pointeur graphique. La zone ciblée Z_{C} peut également être désignée par la définition de coordonnées de l'espace d'une image bidimensionnelle ou tridimensionnelle par exemple à partir d'un logiciel configuré pour piloter le réseau de phase.

Selon un mode de réalisation, la position de la zone ciblée Z_{C} peut être déterminée au moyen d'un cathéter intracardiaque comportant une sonde ultrasonore ou électromagnétique introduite dans un ventricule ou une oreillette du coeur et couplée respectivement à un ou des capteurs de position à ultrasons ou à un ou plusieurs capteurs de position électromagnétiques. Ledit système de positionnement peut donc être associé avec un système d'imagerie IRM ou au moins une sonde ultrasonore ou encore au moins un capteur intracardiaque. Une sonde ultrasonore permet, selon un mode de réalisation, de définir la zone ciblée Z_{C} pour asservir la position du faisceau focalisé et donc de la zone focalisée Z_{F}. Ce système peut être utilisé en complément d'un système d'imagerie IRM ou en substitution de celui-ci pour les mesures de déplacements du tissu.

Le système de positionnement permet de définir une position de la zone ciblée Z_{C} qui est transmise au réseau de phase ou au générateur de signaux ultrasonores pour l'asservir en position. En second lieu et éventuellement, le système de positionnement est capable de récupérer la position de la zone focalisée Z_{F} pour en déduire un écart avec la consigne de position de la zone ciblée. Le système de positionnement peut donc être intégré à un système d'imagerie par une fonction implémentée dans un calculateur ou être externe au système d'imagerie et y être associé pour extraire des données de l'image acquise.

Selon un mode de réalisation, le procédé de l'invention permet d'identifier une ou plusieurs zones ciblées Zc. Cette étape permet de réaliser différentes fonctions dont l'asservissement de la zone focalisée Z_{F}, comme décrit précédemment.

Une fonction assurée par les procédés de l'invention, selon un mode de réalisation, comprend une comparaison des données d'imagerie de la zone ciblée Zc ou d'une zone à sa proximité avant et après l'émission d'une impulsion de contrôle dans le coeur pour déterminer un paramètre de déformation tissulaire et/ou un paramètre de variation de températures. En effet, lorsqu'une comparaison d'images est réalisée, le procédé de l'invention permet d'identifier un gradient de valeurs sur l'image, c'est-à-dire une variation traduisant par exemple un déplacement du tissu cardiaque et/ou une variation de températures. C'est notamment le cas, lorsque le système d'imagerie est un système d'imagerie IRM et qu'il est couplé à un calculateur K₁. Sur la figure 1, une étape VERIF_1 permet de contrôler la calibration de la balistique entre le système d'imagerie et le système de positionnement. Ce contrôle consiste à vérifier que la position d'un point de l'espace évaluée par un équipement corresponde bien à la position d'un point de l'espace évaluée par un autre équipement. Après l'application d'un champ magnétique, ce dernier permet de déduire un paramètre de déphasage comprenant une valeur relative au déplacement des tissus et une valeur relative à la variation de températures. Le calculateur K1 permet d'effectuer notamment des opérations de traitement d'images pour déduire des paramètres de contrôle.

### Réseau de phase, générateur de signaux

Une troisième étape, notée GEN F_{US}, comprend la génération d'un ou d'une pluralité de faisceau(x) de signaux ultrasonores F_{US} focalisé(s) dans la ou les zone(s) ciblée(s) Z_{C}.

La sonde thérapeutique à ultrasons est montée sur un système mécanique de positionnement 3D permettant de localiser la sonde à l'aplomb de la région cardiaque ciblée. Grâce à la technologie réseau de phase, le faisceau ultrasonore peut être défléchi électroniquement autour de la position naturelle du point focal pour un réglage fin de la zone de tir. Cela permet de cibler aisément différentes régions du coeur pour réaliser par exemple l'ablation simultanée de différentes régions du coeur.

Selon un exemple de réalisation, un réseau de phase à 256 éléments peut être utilisé avec une fréquence centrale à 1MHz. Le foyer géométrique peut être configuré à 13cm.

Dans un mode de réalisation, le réseau de phase de signaux ultrasonores focalisé F_{US} comprend un ensemble d'éléments, tels que transducteurs élémentaires. La configuration du réseau de phase permet l'activation ou la désactivation des éléments, et le paramétrage des phases de chaque signal qui permet de piloter la déflexion du faisceau. La position du point focal du faisceau est donc déterminée par le paramétrage des phases de chaque signal définissant une déflexion donnée.

La configuration du réseau de phase permet de prendre en compte des obstacles entre les éléments du réseau et le coeur tels que des côtes ou d'autres organes. Ainsi, il est possible de configurer la génération d'un ou de plusieurs faisceau(x) tout en évitant les obstacles. Cela permet, par exemple, de ne pas endommager les côtes d'un patient. Le procédé d'ablation de l'invention est particulièrement efficace lorsque le réseau est positionné en vis-à-vis de cage thoracique, au plus proche du coeur. Dans cette configuration, le procédé de l'invention permet d'établir une configuration du réseau de phase afin d'éviter d'insonifier des côtes, des brûlures pouvant être induites par absorption d'ondes ultrasonores émises dans une paroi osseuse.

Cette configuration peut être réalisée lors de la calibration d'un faisceau focalisé ou lors de l'opération d'ablation d'au moins une zone du coeur. La déflexion du faisceau peut être asservie pour compenser des mouvements respiratoires ou des contractions du coeur induisant un déplacement de la zone ciblée. Une nouvelle position d'une zone ciblée peut être déterminée par une estimation du déplacement de cette zone grâce à un système de positionnement tel que détaillé précédemment.

Les zones ciblées Z_{C} définissent une région dans le référentiel du coeur et sont donc susceptibles de se déplacer dans un référentiel terrestre. Le réseau de phase RES_US doit donc compenser les mouvements du coeur induits par les mouvements respiratoires ou les contractions du coeur. Ceci est réalisé grâce à l'asservissement de la position focalisée Z_{F} sur la position ciblée Z_{C}.

En particulier, selon un mode de réalisation, la déflexion du faisceau est asservie sur un paramètre de compensation des mouvements respiratoires du coeur. En effet, les mouvements de respirations génèrent des déplacements de la zone ciblée Z_{C} tout au long du cycle cardiaque et sont préférentiellement compensés lors de l'exécution des procédés de l'invention.

Eventuellement, selon un mode amélioré, la déflexion du faisceau peut être asservie sur un paramètre de compensation des mouvements de contraction du coeur survenant lors du complexe QRS. Une alternative est d'éteindre automatiquement le faisceau pendant l'apparition du complexe QRS pour éviter, par exemple, lors du procédé d'ablation d'endommager les tissus d'une zone connexe à la zone ciblée Z_{C} lors d'un mouvement du coeur non compensé. Dans ce cas, l'extinction du faisceau est asservie sur le rythme de l'ECG pour que les moments d'apparition de la contraction, lors du complexe QRS, et de l'extinction du faisceau soit synchrones.

### Définition des signaux

Dans tous les modes de réalisation, le faisceau de signaux ultrasonores focalisé F_{US} est généré à une amplitude et à une durée configurées de manière à contrôler les niveaux de dommages mécaniques et/ou thermiques sur le tissu cardiaque pour calibrer les niveaux de signaux à appliquer lors de l'opération d'ablation.

Une calibration des signaux selon le procédé de contrôle de l'invention peut être réalisée pour préparer une phase d'ablation dans laquelle les signaux définissent un ou plusieurs faisceaux optimisés. L'optimisation est effectuée en calibrant l'amplitude, la durée d'impulsion, le nombre et la durée des microcoupures ainsi que la répétition des impulsions du faisceau focalisé.

Les faisceaux générés par le procédé de contrôle ou le procédé d'ablation sont avantageusement générées de sorte que le front montant de l'impulsion survienne pendant la dépolarisation des tissus cardiaques. La synchronisation avec l'ECG permet donc de s'assurer du bon moment pour générer le faisceau focalisé. La génération du faisceau pendant la dépolarisation des tissus permet d'éviter toute stimulation des tissus pouvant induire une contraction du coeur ou une activité électrique favorisant une telle contraction.

La calibration du réseau de phase RES_US dépend de différents critères pour le contrôle et l'ablation : âge, corpulence du patient, taille du coeur, épaisseur des tissus cardiaques, myocarde, zones stimulées, etc. La calibration du réseau de phase permet donc d'adapter un faisceau pour définir une ablation efficace.

L'ablation peut être réalisée soit par brûlure du tissu cardiaque lorsque la température s'élève au-delà d'un seuil dans la zone focalisée, soit par cavitation inertielle, lorsque le niveau d'amplitude du faisceau appliqué est supérieur à un seuil donné.

Lorsque l'ablation est effectuée principalement en générant des dommages thermiques sur les tissus entrainant une brûlure ou une nécrose des tissus, la durée du signal peut être calibrée pour une amplitude donnée du faisceau.

Lorsque l'ablation est effectuée principalement en générant des dommages mécaniques par la génération d'un phénomène de cavitation inertiel, l'amplitude du faisceau doit être configuré supérieur à un certain seuil.

Lors d'une opération d'ablation, les effets des dommages thermiques et mécaniques apparaissent le plus souvent conjointement. Le procédé d'ablation de l'invention permet une configuration des faisceaux permettant d'obtenir une proportion d'un effet donnée plus significativement vis-à-vis d'un autre effet.

Selon un mode de réalisation, le procédé de l'invention comprend une étape d'injection d'un agent de contraste ultrasonore dans l'organe, c'est-à-dire le coeur.

Selon un premier mode de réalisation, l'agent de contraste ultrasonore permet de visualiser les faisceaux ultrasonores et de suivre le traitement par un système d'imagerie ultrasonore. Selon ce mode de réalisation, l'agent de contraste ultrasonore peut être utilisé en combinaison avec un dispositif d'imagerie ultrasonore de la même manière qu'un agent de contraste est utilisé pour l'amélioration de la visualisation des images IRM. Cet usage permet par exemple de détecter les zones saines et les zones pathologiques.

Ce contrôle peut être effectué :
▪ avant une opération de calibration des signaux focalisés ou avant une opération d'ablation pour préparer l'opération ;
▪ pendant ces opérations par exemple pour réaliser un contrôle actif sur les signaux de manière à les réguler ou à stopper l'émission, ou encore ;
▪ après ces opérations pour vérifier par exemple qu'une ablation a bien été effectuée.

Selon un second mode de réalisation, l'agent de contraste ultrasonore peut être utilisé pour favoriser l'action du faisceau focalisé ultrasonore. La présence de l'agent de contraste permet d'améliorer l'apparition d'un phénomène de cavitation lorsqu'un signal est focalisé par la génération de microbulles. L'apparition de ce phénomène étant favorisée, une conséquence est de réduire le seuil des maximas d'amplitude des signaux acoustiques permettant par exemple l'ablation d'une zone focalisée du coeur. Dans ce contexte, l'agent de contraste ultrasonore permet de réduire les seuils minimaux des niveaux de signaux ultrasonores utilisés pour ablater une zone du coeur. L'agent de contraste ultrasonore permet donc de diminuer les puissances émises par le générateur de faisceaux en obtenant un résultat équivalent de celui obtenu avec une puissance plus forte sans agent de contraste ultrasonore.

Un intérêt de cette solution est de diminuer les puissances émises et donc de réduire les risques de dommages des tissus à proximité de la zone à ablater. Un autre intérêt est de diminuer les dommages causés sur d'autres organes ou sur des os par des émissions du faisceau.

Un dispositif pouvant être utilisé peut être par exemple celui nommé « SonoVue » de Bracco.

L'administration de l'agent de contraste ultrasonore peut être injectée dans une large gamme de valeurs. Des tests effectués sur des coeurs de cochons ont permis de valider l'amélioration d'une ablation d'une zone de 1 à 2 cm³ avec des puissances de pression acoustique plus faibles lorsqu'un agent de contraste ultrasonore était utilisé. Ces données sont d'un même ordre de grandeur que celles pouvant être obtenues chez l'homme. A titre d'exemple, une injection allant de 0,03 à 0,20 ml/kg de SonoVue peut être utilisée pour obtenir un effet d'amélioration de l'effet d'ablation des tissus par application d'un faisceau focalisé. Une valeur de 0,1 ml/kg aboutit à un résultat concluant selon des tests conduits avec des signaux acoustiques focalisés d'une puissance de 200 à 300 W pendant une durée de 10 à 20s.

Dans ce test, la demi-vie d'élimination terminale a été de 12 minutes (allant de 2 à 33 minutes).

L'utilisation d'un agent de contraste ultrasonore permet d'obtenir des réductions de puissances nécessaires dans la zone focalisée allant de quelques centièmes de MPa à quelques MPa. Les gains obtenus dépendent de la quantité d'agent de contraste ultrasonore administrée, de la période à laquelle le faisceau est émis après l'administration de l'agent de contraste et de données physiologiques de l'organe propres au patient ou à l'animal.

Un autre avantage est que l'effet de l'agent de contraste ultrasonore est indépendant de la durée de l'impulsion du faisceau, ce qui garantit un mode opératoire commun. C'est-à-dire qu'il est possible d'obtenir la même amplification des signaux pour différentes durées d'impulsions générées pendant la période pendant laquelle l'agent de contraste produit un effet.

### Contrôle des paramètres

Dans un mode de réalisation, le réseau de phase RES_US est configuré en phase pour générer une pluralité de faisceaux focalisés et donc générer une pluralité d'impulsions sur plusieurs zones ciblées Z_{C}. La stimulation multizone lors du procédé de contrôle de l'invention permet d'observer les réponses électriques et/ou les déformations et/ou les températures desdites zones et ainsi de calibrer les faisceaux pour réaliser une ablation. Des comparaisons des réponses permettent d'étalonner le(s) faisceau(x) focalisé(s) utilisé(s) pour planifier une opération d'ablation.

Lorsque qu'une ablation est réalisée sur une pluralité de zones ciblées Z_{C} cela permet de mutualiser l'opération et donc un gain de temps.

Dans un mode de réalisation, le faisceau de signaux ultrasonores focalisé F_{US} est configuré pour générer une impulsion sur une zone ciblée Z_{C} de sorte à stimuler les tissus cardiaques dans cette zone. Comme précisé précédemment, le procédé de l'invention permet d'analyser la réponse électrique et/ou la déformation tissulaire et/ou la température et/ou un niveau de cavitation dans ou à proximité de ladite zone soit avant l'ablation pour la configuration des faisceaux, soit pendant pour contrôler le bon déroulement de l'ablation.

Un avantage est de permettre un arrêt automatique d'une ablation en cours lorsqu'une erreur de contrôle est détectée. A titre d'exemple, si une erreur est commise, par exemple sur la balistique, le procédé d'ablation peut être automatiquement arrêté. Dans ce cas, le procédé de l'invention est capable de relancer une calibration de la position d'une zone ciblée entre le système de positionnement et le réseau de phase RES_US.

### Contrôle de la température

Le procédé de l'invention permet de contrôler certains paramètres tels que la température ACQ_T en une ou plusieurs zones du coeur pour contrôler lesdites zones lors des opérations de calibration ou d'ablation. Un afficheur AFF_2 permet dans un mode de réalisation de visualiser en coupe ou en 3D les températures du coeur ou d'au moins une région particulière de ce dernier. La figure 1 représente un unique afficheur AFF_2 mutualisant les affichages de différents équipements. Selon un autre mode de réalisation, différents afficheurs peuvent être utilisés et dédiés à chaque équipement dans les procédés de l'invention.

Un calculateur, noté K₁, permet d'extraire les données acquises par un système d'imagerie IMG, tel qu'une IRM, pour déduire les températures dans certaines zones du coeur. Une extraction de la position de la zone ciblée ou de la zone focalisée peut être réalisée pour identifier la température dans cette zone ou à proximité. Par exemple, le calculateur K₁ est capable de calculer des variations de températures par une analyse des données de l'image acquise et d'identifier automatiquement les zones pour lesquelles les variations dépassent un seuil de température. Les positions des zones focalisées peuvent donc être déduites automatiquement par un traitement des données de l'image acquise en IRM.

Lorsque les mesures de températures sont effectuées par un système d'imagerie IRM, la différence intrinsèque entre le déphasage dû à un changement de température et celui dû à un déplacement local, permet de discriminer les variations de températures.

Selon un premier mode de réalisation, le contrôle de la température peut être réalisé lors de l'exécution du procédé de contrôle de l'invention pour calibrer le faisceau focalisé utilisé lors de l'opération de l'ablation.

Selon un second mode de réalisation, le contrôle de la température peut être réalisé lors de l'exécution du procédé d'ablation. Dans ce cas on parle d'un « contrôle actif ». Ce contrôle actif permet notamment de surveiller l'échauffement des zones adjacentes à la zone ciblée pour éviter une surchauffe de zones dans lesquelles les tissus cardiaques ne doivent pas subir de dommage.

Lorsque les variations de températures sont visibles à l'écran, un code couleur peut aider un opérateur à prévenir des hausses importantes de températures.

### Contrôle de l'activité électrique

Dans un mode de réalisation, une mesure de l'activité électrique locale, notée ACQ_AE, peut être effectuée pendant et/ou après la génération du faisceau de signaux ultrasonores focalisé GEN F_{US} en complément de la mesure de la température de manière à vérifier l'efficience de l'ablation dans la zone focalisée et/ou à vérifier que les zones adjacentes répondent électriquement.

Selon un mode de réalisation, le niveau d'activité électrique peut être également mesuré à partir d'un cathéter électrique introduit dans une région du coeur : ventricule ou oreillette. Cela permet de vérifier que la zone ne comprend pas de tissus ne se déformant pas et pouvant être néanmoins conducteurs électriquement.

Selon d'autres modes de réalisation, d'autres équipements peuvent être utilisés permettant de mesurer une activité électrique localement dans ou à proximité de la zone focalisée. Cet équipement est noté SYS-ELECT_2 sur la figure 1. Il permet d'acquérir le signal ACQ_AE traduisant un niveau d'activité électrique local. Un calculateur K2 peut être adjoint au système. Il permet la mise en oeuvre d'un mode de l'invention en comparant un niveau d'activité électrique à un niveau de référence ou encore de rapprocher les valeurs de niveau d'activité électrique avec la mesure de déformation tissulaire ACQ_DEF. Ces corrélations / comparaisons de valeurs sont représentées par la fonction COMP de la figure 1 réalisées par exemple au moyen du calculateur K2. Un message d'état VERIF_2 permet d'aboutir au résultat de la comparaison. Selon un mode de réalisation de l'invention les fonctions du calculateur K2 peuvent être réalisées par le calculateur K1.

Les temps de réponse électrique de chaque zone ciblée Z_{C} peuvent également être mesurés.

Le procédé de contrôle de l'invention permet à partir d'une impulsion de calibration de définir les seuils de stimulations électriques permettant de causer des dommages aux tissus afin de les nécroser ou les détruire. Il est possible de déduire un niveau d'activité électrique théorique généré en fonction d'un niveau mesuré de déformation des tissus.

Lorsque l'activité électrique de la zone focalisée est inférieure à un seuil prédéterminé, la zone focalisée est alors considérée comme « non répondante » et l'opération d'ablation peut se terminer. Lorsque la zone focalisée est considérée comme non répondante, le procédé de contrôle ou le contrôle actif pendant le procédé d'ablation permet de vérifier que l'ablation d'une zone génératrice d'une arythmie s'est bien déroulée et est terminée.

Le procédé de contrôle peut favoriser un étalonnage permettant d'établir une règle de correspondance entre les niveaux d'activités électriques et les niveaux de déplacements des tissus mesurés lors de l'application d'un faisceau focalisé dans une zone focalisée donnée. Cet étalonnage peut également prendre en compte les réponses aux températures dans cette zone. La corrélation permet notamment de déterminer un indicateur d'activité mécano-électrique du tissu cardiaque d'une zone ciblée donnée.

Lors du procédé de contrôle ou d'ablation, cet étalonnage peut permettre de mesurer uniquement les niveaux de déformation des tissus et déduire l'activité électrique induite et de configurer le faisceau avec une amplitude et une durée adéquate pour procéder à l'ablation de la zone ciblée.

Lorsque la mesure de la déformation des tissus, ou d'une caractéristique propre à leur élasticité, est faible, voire nulle, alors il est possible de déduire qu'aucune activité électrique est générée. Les procédés de l'invention peuvent donc être avantageusement non invasifs.

Notons qu'une activité électrique ACQ_AE locale dans la zone ciblée peut être contrôlée et déduite de l'ECG obtenu par exemple au moyen des électrodes. Dans ce dernier cas de figure, les systèmes électriques SYS_ELEC_1 et SYS_ELEC_2 de la figure 1 peuvent ne former qu'un seul équipement. Notamment, lorsqu'une impulsion est générée par le procédé de contrôle de l'invention et qu'une dépolarisation globale d'un ventricule ou d'une oreillette a lieu, alors l'activité électrique induite par le procédé de contrôle est visible sur un ECG par la présence d'un pic. Dans ce cas, un cathéter électrique peut éventuellement être utilisé pour corréler les mesures d'activités électriques déduites de l'ECG ou pour obtenir des mesures plus précises.

Ce moyen peut permettre de détecter rapidement des zones supposées non répondantes qui sont ensuite testée localement électriquement ou mécaniquement.

### Contrôle d'une présence d'une contraction cardiaque

Enfin, les procédés de contrôle et d'ablation de l'invention peuvent comprendre une étape de vérification de la présence d'une contraction cardiaque. La vérification d'une contraction mécanique peut être effectuée au moyen d'une sonde de pression sanguine par exemple par une mesure du pouls ou par une sonde intra-aortique pour mesurer la contraction du coeur. Cet équipement de mesure de contraction mécanique est noté CAP_CONTRACT sur la figure 1 et la mesure est notée ACQ_CONTRACT.

### Contrôle de la cavitation

Selon un mode de réalisation, le procédé de contrôle ainsi que le procédé d'ablation de l'invention comprennent une étape de calcul d'un niveau de cavitation dans la zone focalisée. Le phénomène de cavitation, repose sur le phénomène de création de bulles dans la région focalisée générée par la vibration des ondes ultrasonores émises.

On distingue deux cavitations pouvant survenir :
▪ une cavitation stable qui correspond à un phénomène de création de bulles dans la zone focalisée ou à proximité. Les bulles favorisent éventuellement le déplacement des tissus;
▪ une cavitation inertielle qui est dans la continuité de la cavitation stable dans laquelle les bulles se vaporisent ou éclatent pouvant créer des dommages sur les tissus mais pouvant également stimuler la zone. Le phénomène de cavitation inertielle est créé par génération d'une pression négative localement au-delà d'un certain seuil.

Le phénomène de cavitation peut être déterminé par un dispositif de mesure d'un niveau de cavitation. Selon un exemple de réalisation, il peut s'agir d'un dispositif ultrasonore pour la détection d'un niveau de cavitation ACQ_CAV dans ou à proximité de la zone focalisée, noté SYS_CAV sur la figure 1. Ce dispositif comprend un capteur ou plusieurs capteurs d'ondes ultrasonores et un calculateur effectuant une analyse spectrale ACQ_US des ondes ultrasonores réfléchies dans ou à proximité de la zone focalisée.

Selon un mode de réalisation, un calculateur K1 permet de centraliser les différentes mesures effectuées par différentes équipements dont les mesures de niveau de cavitation ACQ_CAV, de déformation tissulaire ACQ_DEF, de température ACQ_T. Les mesures sont éventuellement comparées à des seuils. Des alarmes peuvent être générées selon les dépassements vis-à-vis des seuils prédéfinis de ces mesures en temps réel. Un afficheur AFF_2 permet éventuellement d'afficher ces valeurs et les images acquises par les différents équipements.

Selon un autre mode de réalisation, chaque équipement peut être couplé à un calculateur dédié et un afficheur dédié.

Plus le spectre est étalé, c'est-à-dire que le bruit est important dans ou à proximité de la zone focalisée, plus le phénomène de cavitation est déterminé comme important. Des seuils peuvent être définis et déterminés à partir du procédé de contrôle de l'invention pour étalonner les valeurs d'amplitude et de durée des signaux utilisés notamment pour l'ablation. Selon un mode de réalisation, le contrôle actif effectué pendant le procédé d'ablation détecte en temps réel un niveau représentant l'importance du phénomène de cavitation. Le procédé d'ablation peut prendre en compte dynamiquement les niveaux mesurés :
▪ soit pour stopper le ou les faisceaux en cas de niveau trop important ;
▪ soit pour configurer automatiquement le niveau de pression générée au point focal par une consigne d'amplitude ou encore pour configuré la durée d'application restante du faisceau.

Selon un premier mode, le procédé d'ablation est configuré avec un faisceau dont l'amplitude est inférieure à un premier seuil de cavitation et dont la durée est supérieure à la seconde. Les niveaux d'amplitude du faisceau peuvent être configurés pour une pression appliquée au point focal de 2 à 5 MPa. Dans ce mode de réalisation, le procédé d'ablation est configuré pour éviter la formation d'un phénomène de cavitation stable, ce phénomène pouvant favoriser la stimulation du coeur, ce qu'on cherche à éviter. En effet, un danger de la stimulation du coeur pendant le procédé d'ablation est de favoriser la survenance d'une dépolarisation électrique pouvant causer un problème d'asservissement balistique par exemple. Ainsi, le procédé d'ablation de l'invention comprend une configuration dans laquelle, la zone ciblée est endommagé préférentiellement par la création de dommages thermiques, c'est-à-dire grâce à des impulsions de longues durées et avec des amplitudes de signaux maîtrisées.

Le faisceau est préférentiellement généré avant l'onde T, par exemple dans l'intervalle QT de l'ECG. Dans ce cas, le faisceau avantageusement ne crée pas de stimulation électrique dans la zone ciblée du coeur.

Selon un second mode, le procédé d'ablation est configuré avec un faisceau dont l'amplitude est élevée, de 1 à 100MPa pendant des impulsions de courtes durées. Les durées d'application du faisceau sont de l'ordre de quelques millisecondes correspondant à quelques cycles de vibrations ultrasonores. Une gamme de valeurs temporelles est de 1ms à 50ms. Dans un mode préféré, des durées d'impulsion de 5ms à 10ms permettent de minimiser l'apparition du phénomène de cavitation stable et inertielle. Un exemple d'amplitude de 8 à 12 MPa avec des impulsions de 5 à 10ms permettent d'ablater une zone ciblée tout en évitant de stimuler électriquement la zone focalisée.

Dans ce mode de réalisation, le procédé d'ablation est configuré pour éviter la formation d'un phénomène de cavitation inertielle favorisant la stimulation du coeur et les dommages des tissus cardiaques dans ou à proximité de la zone ciblée. En effet, un danger de la stimulation du coeur pendant le procédé d'ablation est de favoriser la survenance d'une dépolarisation électrique pouvant causer un problème d'asservissement balistique par exemple. Ainsi, le procédé d'ablation de l'invention comprend une configuration dans laquelle, la zone ciblée est endommagé préférentiellement par la création de dommages mécaniques, c'est-à-dire au moyen d'impulsions de courtes durées avec une intensité de force de radiation locale importante.

Le faisceau est préférentiellement généré avant l'onde T, par exemple dans l'intervalle QT de l'ECG. Dans ce cas, le faisceau avantageusement ne crée pas de stimulation électrique dans la zone ciblée du coeur.

Dans un troisième mode de réalisation, le procédé d'ablation est réalisé en maitrisant le phénomène de cavitation inertielle provoquant des dommages sur les tissus. En effet, l'ablation peut être réalisée en maitrisant les dommages des tissus crées par le phénomène de cavitation inertielle. En revanche, un risque de ce mode de réalisation est de générer une stimulation d'une zone du coeur pouvant perturber le bon déroulement du procédé d'ablation, notamment par la survenance d'une contraction du coeur causant une erreur sur la balistique. Pour limiter ce risque, les impulsions sont générées lors de la dépolarisation des tissus cardiaques, par exemple pendant la période QT de l'ECG pour le ventricule.

Selon un autre mode de réalisation qui peut se combiner avec les précédents modes, une mesure d'un niveau de cavitation est comparée à un premier seuil et une mesure d'une déformation tissulaire est comparée à un second seuil.

Selon une première variante, les seuils peuvent être des seuils qui correspondent à la détection de risques de dommages des tissus d'une zone voisine à la zone focalisée. En effet, lors d'une opération d'ablation, il peut être nécessaire de contrôler et de s'assurer que des zones limitrophes à la zone d'ablation ne subissent pas de dommages. Le franchissement du seuil peut donc indiquer un risque de dommage sur une zone limitrophe à la zone à ablater.

Selon une seconde variante qui peut se combiner avec la première variante lorsque différentes zones sont contrôlées, les seuils peuvent être des seuils qui correspondent à un indicateur de nécrose représentant par exemple un pourcentage de nécrose d'une zone à ablater. Le franchissement du seuil peut donc indiquer un temps d'application restant du signal focalisé pour terminer l'ablation.

Un algorithme permettant de prendre en compte les risques de dommages voisins ou le taux de nécrose d'une zone à ablater permet de générer une consigne d'arrêt de la procédure d'ablation, par exemple en coupant automatiquement le signal ultrasonore émis. Un intérêt est de renforcer la procédure de sécurité encadrant une intervention visant à ablater une one tout en s'assurant que les zones voisines restent saines.

Enfin, un autre intérêt réside dans la possibilité d'obtenir un double indicateur dans le temps basé sur une double appréciation par deux moyens différents soit d'un risque de dommage, soit d'un indicateur de nécrose. En effet, un capteur ultrasonore mesurant le niveau de cavitation peut acquérir entre 10 et 5000 signaux par seconde, alors qu'un capteur de déformation des tissus, utilisant par exemple l'IRM, nécessitera un taux de rafraichissement de son acquisition de 0,5 à 10 signaux par seconde. Il est donc possible de disposer d'un système comprenant des moyens de calculs permettant d'effectuer une première mesure du niveau de cavitation et de comparer cette mesure à un seuil. Une seconde mesure d'un niveau de déformation peut être effectuée successivement à la mesure de cavitation. La détection de l'évolution du niveau de déformation peut être analysée dans l'espace de temps succédant la détection du premier risque identifié. De ce fait, il est possible d'engendrer une coupure du signal focalisé le plus rapidement possible pour des raisons de sécurité si la zone contrôlée est une zone voisine à la zone focalisée ou d'effectuer une simple mesure dans la zone focalisée pour estimer le temps restant à terminer une ablation.

Par ailleurs, dans la zone focalisée ou dans une zone voisine, le premier dépassement du niveau de cavitation peut conduire à analyser l'évolution de la déformation tissulaire sans que pour autant le second seuil ne soit dépassé. L'analyse porte alors sur la tendance des tissus à se déformer et non sur le dépassement d'un second seuil donné. Ainsi, il est possible grâce au procédé de l'invention de couper ou de maintenir la génération du signal en fonction de l'analyse des mesures.

Selon une alternative, la mesure du niveau de cavitation n'est plus corrélée avec la mesure de déformation tissulaire mais avec l'activité électrique dans ou à proximité de la zone focalisée. Dans ce dernier cas, le même traitement peut être appliqué à ces deux mesures, notamment en ce qui concerne l'analyse des signaux par les différents équipements dans le temps.

L'algorithme peut également permettre la génération d'une coupure des signaux ultrasonores uniquement lorsque deux seuils sont dépassés lors d'un contrôle d'une zone voisine. Un premier seuil concerne le niveau de cavitation et un second seuil concerne, par exemple, la déformation des tissus. Cette option permet de considérer des erreurs de mesures d'un des appareils de mesure, tels que l'IRM ou un dispositif ultrasonore par exemple.

Le procédé de l'invention permet donc de définir différentes stratégies de contrôle d'une zone voisine à une zone à ablater ou de la zone à ablater.

Une première stratégie peut correspondre à assurer un degré de sécurité maximale. Dans ce cas, un seul dépassement de seuil dans une zone voisine permet de générer une coupure du faisceau de signaux. Une seconde stratégie correspond à assurer l'élimination des erreurs de mesures. Dans ce cas, le procédé permet de confirmer qu'un dommage survient dans une zone voisine si deux seuils de mesures d'équipements différents sont dépassés.

Notons que le procédé s'applique avec une pluralité de mesures correspondantes à des grandeurs mesurées avec différents équipements. Ainsi, une corrélation de trois types de signaux peut être effectuée en effectuant des comparaisons vis-à-vis de trois seuils. Par exemple, le niveau de cavitation est comparé à un premier seuil, une déformation tissulaire est comparée à un second seuil et une activité électrique est comparée à un troisième seuil. Une stratégie de contrôle d'une zone voisine peut être que le dépassement de deux seuils sur trois suffit à générer une coupure des signaux ultrasonores. Cette solution permet un compromis entre un gain de sécurité (2 appareils sur 3 ont détecté un risque) et une prise en compte d'erreurs de mesure (1 appareil sur 3 n'a rien détecté). D'autres possibilités peuvent être décidées selon si l'on souhaite une configuration favorisant une sécurité maximale : au moins un seuil est dépassé entraine la coupure du faisceau ou selon si l'on souhaite une configuration dans laquelle on prend en compte des erreurs de mesures : les trois seuils doivent être dépassés pour engager une coupure du faisceau.

### Contrôle de la déformation tissulaire

La déformation tissulaire peut être mesurée localement lors de l'exécution du procédé de contrôle pour vérifier un état d'élasticité du tissu cardiaque. La déformation tissulaire peut être utilisée pour apprécier les caractéristiques des tissus d'une zone causant une arythmie cardiaque et donc de valider l'opération d'ablation de la zone ciblée.

La génération du ou des faisceau(x) de signaux ultrasonores GEN F_{US} permet de générer une poussée ultrasonore localisée dans une zone focalisée Z_{F} et possiblement sensiblement à proximité de cette zone par déplacement des tissus.

Dans le cas de l'ablation, la mesure de la déformation des tissus localement sert à calibrer le niveau de pression au moyen du procédé de contrôle de l'invention et à vérifier la balistique sans induire d'effet thermique.

En effet, lorsque la déformation tissulaire est repérée par un système d'imagerie, soit par IRM soit par ultrasons, il est possible de corréler les données de positions avec celles du système de positionnement. Typiquement, les zones dans lesquelles la réponse élastique des tissus est comparée avec la position ACQ_POS de la zone ciblée identifiée avec le système de positionnement SYS_POS. Cette comparaison permet également de calibrer les positions repérées entre le système d'imagerie, IRM ou ultrasons, et le réseau de phase guidé en position par le système de positionnement.

Enfin, la mesure de la déformation des tissus permet de calibrer les niveaux d'élasticité du tissu dans une zone ciblée pendant le procédé d'ablation pour constater qu'une nécrose de coagulation est terminée, par exemple, dans la zone ciblée. Une comparaison des niveaux d'élasticité pendant l'ablation avec une mesure réalisée préalablement permet de quantifier la proportion de zone ablatée en fonction d'un niveau à atteindre ou encore de quantifier la durée restante d'application du faisceau.

Les déformations tissulaires peuvent être mesurées à partir d'un système d'imagerie IRM comme précisé précédemment. D'autres systèmes d'imagerie peuvent être utilisés lorsque ces derniers permettent de contrôler une déformation ou une élasticité du tissu. Selon un autre mode de réalisation, les déformations tissulaires peuvent être mesurées par un cathéter comprenant une sonde à ultrasons introduite à proximité de la zone focalisée (Z_{F}) mesurant la déformation tissulaire. Eventuellement, un cathéter comprenant une sonde de pression peut être utilisé à proximité de la zone focalisée pour déduire des déformations tissulaires.

Le phénomène de cavitation dans la zone ciblée peut être également mesuré par les procédés de l'invention :
▪ soit par extrapolation d'une déformation tissulaire essentiellement générée par ce phénomène ;
▪ soit par des capteurs à ultrasons extracorporels comme précisé précédemment par une analyse spectrale des signaux réfléchis ;
▪ soit par un capteur ultrasonore intracardiaque positionné à proximité de la zone ciblée.

### Phénomène de destruction des tissus cardiaques

Ainsi le procédé d'ablation de l'invention peut être configuré de sorte à générer des dommages sur les tissus cardiaques dans une zone focalisée de sorte à les nécroser ou les brûler.

Les dommages peuvent être causés :
▪ par un phénomène de brûlure par un échauffement des tissus au-delà d'un certain seuil de température, par exemple avec des impulsions dont l'amplitude est limitée et dont la durée d'application est suffisamment longue pour produire un échauffement de la zone considérée ;
▪ par un phénomène de dommages mécaniques en configurant des impulsions très courtes avec d'amplitudes élevées ;
▪ par un phénomène de cavitation inertielle obtenu pour une certaine puissance ou une amplitude donnée du faisceau focalisée.

Les trois phénomènes peuvent se combiner de sorte à créer des dommages thermiques et mécaniques et par cavitation inertielle sur les tissus cardiaques. Le procédé d'ablation de l'invention permet une configuration ayant pour objectif de maitriser les dommages liés à chaque phénomène notamment pour éviter :
▪ d'endommager les zones voisines,
▪ d'endommager plus que nécessaire les zones ciblées,
▪ une génération d'une stimulation cardiaque lors du procédé d'ablation générant une contraction cardiaque ou modifiant la fréquence cardiaque ;
▪ la perte d'asservissement balistique pouvant causer la brûlure d'autres zones que celle concernée par l'ablation ;
▪ de brûler les os de la paroi transcostale.

Des tests ont permis de valider le procédé de l'invention sur des coeurs de porcs, d'un poids de 45Kg+/-5Kg, qui battaient pendant l'opération d'ablation. Selon cet exemple, il a été utilisé un réseau de phases comprenant un réseau de 256 cellules, c'est-à-dire de 256 transducteurs. Une configuration a pu être obtenue pour une focale de 13 m et une ouverture de 13cm. Les émissions ont été réalisées dans un champ magnétique de 1,5 Tesla. Selon cette expérience, grâce au procédé de l'invention, il a été testé qu'avec une puissance de 300 W dans la zone focalisée avec une durée d'impulsion de 15s, il est alors possible d'obtenir une ablation d'une zone du coeur de 1 à 2cm³. Une telle ablation peut être réalisée à l'intérieur de l'organe à plusieurs millimètres ou centimètres de la paroi du myocarde. On constate que l'élévation maximale de température a été de l'ordre de 21°C+/-1,1°C dans ou à proximité de la zone focalisée.

Une pluralité de zones du coeur peut être ablatée :
▪ soit successivement par une succession d'impulsions dirigées dans différentes zones en calibrant une durée d'impulsion sensiblement égale pour chaque tir, ou ;
▪ simultanément lorsque trois zones focalisées sont pointées par le réseau de phases du générateur de faisceaux.

### Description des figures 2A à 2C

La figure 2A est un diagramme représentant une impulsion pour le contrôle d'une zone ciblée. L'impulsion générée par le faisceau focalisé F_{US} a une amplitude A₁ et une durée D₁, la durée d'application totale étant appelée D_{SIGNAL}. L'amplitude A₁ du signal est représentative de la force de radiation ultrasonore s'exerçant sur les tissus de la zone focalisée.

Les amplitudes Aᵢ et les durées Dᵢ des impulsions générées dans la zone ciblée Z_{C} du coeur sont choisies de manière à optimiser les dommages mécaniques et thermiques tout en préservant les zones limitrophes.

L'impulsion représentée à la figure 2A peut être utilisée à différentes fins :
▪ une calibration du faisceau pour réaliser une opération d'ablation sans dommages voisons à la zone ciblée Z_{C} ;
▪ une calibration de la balistique permettant de calibrer le réseau de phase RES_US et le système de positionnement sur un même référentiel de position.

Dans un mode de réalisation, une impulsion est appliquée sur une zone ciblée Z_{C} du coeur pendant la repolarisation du tissu cardiaque lors de l'étape de contrôle pour la calibration du faisceau de signaux ultrasonores F_{US} dans le but de mesurer l'activité électrique du coeur et/ou la déformation tissulaire et/ou la température induites par l'impulsion dans la zone ciblée Z_{C} du coeur. Selon la réponse électrique, la déformation et/ou la température mesurées, une calibration du faisceau de signaux ultrasonores F_{US} est réalisée.

Dans un autre mode de réalisation, une impulsion est appliquée sur une zone ciblée Z_{C} du coeur pendant la dépolarisation du tissu cardiaque pour le contrôle de la balistique sans générer d'extrasystole. Cela permet de vérifier que la zone focalisée Z_{F} et la zone ciblée Z_{C} du coeur sont confondues ou sensiblement proches.

Les figure 2B et 2C sont des diagrammes, respectivement 31 et 32, représentant deux exemples de faisceaux pour l'ablation d'une zone ciblée Z_{C} du coeur. Lesdites ablations sont réalisées au moyen d'au moins un faisceau de signaux ultrasonores F_{US} appliqué à une amplitude A₁ et pendant une durée d'application totale D_{SIGNAL}. La durée d'application totale D_{SIGNAL} comprend une microcoupure, appelée M_{C}, dans le faisceau de la figure 2B et 5 microcoupures dans le cas de la figure 2C.

Dans les modes de réalisation 31 et 32, un faisceau focalisé F_{US} est configuré de sorte que la pression acoustique A₁ dans la zone focalisée Z_{F} est comprise dans une gamme de pression de [1 - 10 MPa] pour une durée d'application totale D_{SIGNAL} comprise dans la gamme de durées [1 - 120s]. Une durée plus importante peut également être configurée.

Dans ce cas la durée d'application du signal est entrecoupée d'au moins une microcoupure M_{C} comprise dans la gamme de durées [1 ms - 1 s]. La durée d'application D_{SIGNAL} étant la somme des durées D₂ et de la durée des microcoupures M_{C}. La durée des microcoupures est configurée pour permettre la réalisation d'un contrôle actif par exemple au moyen d'une imagerie IRM.

Dans tous les modes de réalisation, les durées des microcoupures M_{C} sont configurées pour réaliser un contrôle de zone ciblée Z_{C}. Notamment les dommages mécaniques et/ou thermiques de la zone focalisée Z_{F} peuvent être déduits d'une mesure de déformation des tissus ou de l'activité électrique dans la zone focalisée. Par exemple, ces mesures, comme précisé précédemment, peuvent être réalisées au moyen d'un système d'imagerie IRM ou d'un cathéter comprenant une sonde ultrasonore ou d'un cathéter électrique.

Une mesure de la déformation des tissus permet de déduire par exemple une mesure de l'élasticité du tissu ou de la durée de relaxation du tissu ou encore sa contraction.

En outre, une mesure de la température dans la zone focalisée permet d'évaluer la présence probable d'un dommage thermique si cette dernière est supérieure à un seuil prédéfinie indiquant une probabilité de dommages thermiques.

Dans un mode de réalisation, l'évaluation des niveaux de dommages mécaniques et/ou thermiques peut être utilisée pour sélectionner l'amplitude et la durée d'application du faisceau de signaux ultrasonores F_{US} pour poursuivre l'ablation de la zone ciblée Z_{C}. Ainsi, suite à une microcoupure M_{C}, la pression acoustique ou la durée pendant laquelle le faisceau de signaux ultrasonores F_{US} est appliqué peuvent être ajustées en augmentant ou au contraire en diminuant lesdites pressions acoustiques et/ou lesdites durées d'application jusqu'à la microcoupure M_{C} suivante.

Il est possible de choisir des impulsions plus courtes et favorisant l'apparition principalement du phénomène de cavitation pour localiser plus précisément les dommages lors de la fin de l'ablation. Cette solution permet de diminuer la durée d'application du faisceau pour limiter les dommages dans des zones limitrophes de la zone ciblée.

Dans tous les modes de réalisation, un contrôle actif de la zone ciblée Z_{C} peut être réalisé par une mesure de l'activité électrique et/ou une mesure de la déformation tissulaire et/ou une mesure de la température de la zone ciblée Z_{C} du coeur de manière à ne pas endommager la zone ciblée Z_{C}.

Dans tous les modes de réalisation, un contrôle actif des zones voisines de la zone focalisée Z_{F} peut être réalisé par une mesure de l'activité électrique et/ou une mesure de la déformation tissulaire et/ou une mesure de la température des zones voisines de la zone ciblée Z_{C} du coeur de manière à ne pas endommager les zones voisines de la zone ciblée Z_{C}.

En effet, lorsque l'activité électrique et/ou la déformation tissulaire et/ou la température de la ou des zones voisines dépasse(nt) un seuil prédéterminé, le faisceau de signaux ultrasonores est configuré pour :
▪ soit diminuer la puissance du faisceau, c'est-à-dire son amplitude engageant une diminution de la pression acoustique, de l'apparition du phénomène de cavitation inertielle et de la température dans la zone focalisée ;
▪ soit suspendre le faisceau automatiquement.

La figure 3 comprend un graphique représentant l'évolution de la température 60 de la zone ciblée Z_{C} en fonction du temps lorsqu'une impulsion 61 d'amplitude A et d'une durée D est générée localement par la formation d'un faisceau focalisé F_{US}.

Selon un mode de réalisation, le faisceau de signaux ultrasonores F_{US} est appliqué sur une zone ciblée Z_{C} du coeur à une amplitude A comprise entre [1 - 10MPa] pendant une durée D_{SIGNAL} supérieure à 1s.

Avantageusement, la durée du signal est comprise entre 1s et 600s. Selon un mode de réalisation, la durée du signal est comprise entre 1s et 120s. Un nombre N de microcoupures peut être programmé de sorte à désactiver la génération du faisceau focalisé pendant cette période de manière à mesurer des paramètres des tissus dans la zone focalisée et à effectuer des calculs.

A l'issue de la durée D d'application du faisceau, la température de la zone ciblée Z_{C} mesurée est contrôlée et maintenue sensiblement à une température T_{ABLATION}. Cette température permet de détruire ou nécroser les tissus cardiaques dans la zone focalisée tout en restant inférieure à une température seuil T_{MAX} pour laquelle une destruction de tissus voisins est observée.

Le procédé de contrôle de l'invention permet partir des mesures effectuées de déduire les températures T_{MAX} et T_{ABLATION} pour un faisceau focalisé donné. Ces dernières déductions peuvent être obtenues au moyen d'abaques ou de tables de correspondances entre :
▪ d'une part, un niveau de déformation des tissus ou un niveau d'activité électrique mesuré observé lors du procédé de contrôle et ;
▪ d'autre part, des niveaux de températures mesurées dans la zone focalisée avant l'ablation observés lors du procédé de contrôle.

La figure 4 est un diagramme représentant un exemple d'ablation d'une zone ciblée Z_{C} du coeur au moyen d'un faisceau focalisé F_{US} appliqué à des amplitudes et à des durées qui sont choisies en fonction de l'élasticité de la zone ciblée Z_{C} mesurée pendant les microcoupures Mc(1), Mc(2). Lesdites microcoupures sont d'une durée supérieure à 1 ms. Selon les calculs effectués sur certains paramètres : température, déformation tissulaire, niveau de cavitation, la durée de la microcoupure peut être configurée pendant une durée plus longue, par exemple de 5ms ou de 10ms. Des microcoupures plus longues peuvent également être réalisées.

Dans un mode de réalisation, un premier faisceau de signaux ultrasonores F_{US} est généré à une amplitude A₁ pendant une durée D₁.

A l'issue de D₁, une première microcoupure M_{C}(1) est effectuée pendant laquelle un calculateur, noté K, relié à un système de mesure de la déformation tissulaire et de la température. A titre d'exemple, une sonde de pression et/ou un système d'imagerie peuvent être utilisés. Les mesures de déformation tissulaire dom(M) et éventuellement de température dom(T°) des tissus de la zone focalisée Z_{F} sont déterminés pour le calcul d'un niveau d'élasticité des tissus dans la zone focalisée Z_{F}.

On note que le calculateur de la figure 4 peut correspondre selon un mode de réalisation au calculateur K1 de la figure 1 lorsque tous les calculs sont mutualisés sur un même calculateur.

A partir de la mesure de l'élasticité des tissus de ladite zone focalisée Z_{F}, le calculateur K détermine automatiquement une donnée correspondant à une amplitude A₂ et une durée d'application D₂ pour l'application d'un second faisceau focalisé F_{US} dans la zone focalisé Z_{F}. La génération du faisceau par le réseau de phase est noté GEN F_{US} sur la figure 4.

De plus, le calculateur K1 peut, avantageusement, déterminer automatiquement l'instant auquel aura lieu une nouvelle microcoupure M_{C}(2) selon le niveau d'élasticité mesuré à la précédente microcoupure Mc(1).

Sur la figure 4, est notée la fonction de génération d'une microcoupure GEN Mc(2).

A l'issue de la durée D₂ de la seconde impulsion du faisceau focalisé, une microcoupure M_{C}(2) peut être effectuée pour mesurer à nouveau des paramètres relatifs aux propriétés des tissus dans la zone focalisée Z_{F}. Dans cet exemple, la seconde microcoupure M_{C}(2) est plus longue que la première microcoupure Mc(1). Selon un mode de réalisation, une mesure du niveau de cavitation est réalisée pendant cette seconde microcoupure Mc(2).

Par exemple, cette dernière mesure de cavitation peut être pilotée:
▪ par une mesure de la température lors de la première microcoupure Mc(1) permettant de générer un indicateur de surveillance du niveau de cavitation à la prochaine microcoupure Mc(2) ;
▪ dès lors que le niveau d'amplitude, c'est-à-dire de l'intensité de la force de radiation générée dans la zone focale, dépasse un certain seuil.

Selon un autre mode de réalisation, la mesure du niveau de cavitation peut être générée automatiquement à chaque microcoupure.

Dans l'exemple de la figure 4, à la fin de la seconde microcoupure Mc(2), si les mesures d'élasticité du tissu indiquent que le tissu a encore des propriétés élastiques au-delà d'un certain seuil au moyen des calculs effectués par le calculateur K, alors l'ablation n'est pas terminée. Dans ce cas, le procédé d'ablation génère, GEN F_{US}, un troisième faisceau de signaux ultrasonores F_{US} d'amplitude A₃ et de durée D₃ après la fin de la microcoupure M_{C}(2). Les niveaux d'amplitude A3 et de durée D3 peuvent être estimés pour finaliser l'ablation à l'issue de ce troisième faisceau.

Cette opération peut être renouvelée jusqu'à ce qu'un niveau d'élasticité seuil des tissus soit calculé. Dans ce cas, le procédé d'ablation de la zone ciblée Z_{C} peut se terminer.

Durant tout le procédé d'ablation, un asservissement de la position du faisceau sur la zone ciblée a été réalisé. Sur la figure 4, n'est pas représenté par exemple les coupures du faisceau lorsque les mouvements de contractions du coeur sont pris en compte pour activer et désactiver le faisceau. Dans ce cas de figure, on suppose que le réseau de phase RES_US est constamment asservi sur les mouvements du coeur dont les mouvements de respirations et les mouvements de contraction.

## Revendications

1. Système pour l'ablation ou le contrôle d'une zone du coeur par ultrasons comprenant :
▪ un moyen de mesure de l'activité électrique du coeur pour l'acquisition d'au moins une fréquence d'un électrocardiogramme (ECG);
▪ un réseau de phase couplé au moyen de mesure de l'activité électrique du coeur pour la génération en synchronisation sur la fréquence acquise de l'électrocardiogramme du coeur (ECG) d'un faisceau de signaux ultrasonores focalisé (Fus) sur au moins une zone ciblée (Z_{c}) du coeur ;
▪ un système de positionnement couplé au moyen de mesure de l'activité électrique du coeur et couplé au réseau de phase (RES_US) de manière à asservir la position d'au moins une zone focalisée d'au moins un faisceau de signaux ultrasonores focalisé (Fus) sur la position de la au moins une zone ciblée (Z_{c}),
▪ un système d'imagerie IRM couplé au moyen de mesure de l'activité électrique du cœur et configuré pour réaliser un contrôle actif et dynamique d'une température et d'une déformation tissulaire dans la zone ciblée (Zc) et/ou dans les zones voisines de la zone ciblée (Zc) ;
▪ un dispositif de mesure d'un niveau de cavitation dans la zone ciblée (Zc)- ;
▪ un calculateur configuré pour ajuster dynamiquement, pendant la génération du faisceau de signaux ultrasonores (FUS) , les caractéristiques dudit faisceau de signaux ultrasonores (FUS) en fonction du niveau de cavitation et en fonction de la température et/ou de la déformation tissulaire dans la zone focalisée en réponse à la génération du faisceau de signaux ultrasonores (FUS)

2. Système selon la revendication 1 dans lequel le dispositif de mesure d'un niveau de cavitation comprend un ou plusieurs capteurs d'ondes ultrasonores et dans lequel le calculateur effectue une analyse spectrale ACQ US des ondes ultrasonores réfléchies dans ou à proximité de la zone focalisée (ZF) pour ajuster dynamiquement, pendant la génération du faisceau de signaux ultrasonores (FUS), les caractéristiques dudit faisceau de signaux ultrasonores (FUS) en fonction du niveau de cavitation.

3. Système selon l'une quelconque des revendications précédentes **caractérisé en ce que** la génération du faisceau de signaux ultrasonores (FUS) est synchronisée sur au moins une fréquence acquise de l'électrocardiogramme (ECG) du coeur.

4. Système selon l'une quelconque des revendications précédentes **caractérisé en ce que** le réseau de phase génère le faisceau de signaux ultrasonores (FUS) avant l'onde T de l'électrocardiogramme.

5. Système selon l'une quelconque des revendications précédentes **caractérisé en ce que** le réseau de phase génère un faisceau de signaux ultrasonores (FUS) une amplitude continue et comprise dans la gamme de valeurs [1-100 MPa] et une durée d'application sur une période supérieure à 1 ms.

6. Système selon l'une quelconque des revendications précédentes **caractérisé en ce que** le système de positionnement et le système de contrôle capable de mesurer une température et une déformation tissulaire dans la zone ciblée (Zc) emploie un même système d'imagerie, ledit système d'imagerie étant un système d'imagerie IRM.

7. Système selon l'une quelconque des revendications précédentes **caractérisé en ce que** le calculateur est configuré pour calculer, pendant la génération du faisceau de signaux ultrasonores, un indicateur soit d'un dommage, soit d'une nécrose de la zone ciblée (Zc), l'indicateur étant calculé en comparant une mesure d'un niveau de cavitation à un premier seuil et en comparant une mesure d'un niveau de déformation à un second seuil.

8. système selon l'une quelconque des revendications précédentes **caractérisé en ce que** les signaux du faisceau de signaux ultrasonores (FUS) sont configurés en phase pour générer au moins une impulsion dans une zone focalisée (ZF), et **en ce qu'**une comparaison de la position de la zone focalisée (Z_{F}) déterminée par le système d'imagerie et de la position de la zone ciblée (Z_{c}) déterminée par le système de positionnement génère au moins une donnée pour calibrer des éléments du réseau de phase de manière à faire correspondre une position de la zone focalisée avec la position de la zone ciblée.

9. Système selon l'une quelconque des revendications précédentes **caractérisé en ce que** l'asservissement dynamique de la position de la zone focalisée (Z_{F}) sur la position de la zone ciblée (Zc) au moyen du système de positionnement (SYS POS) permet de mesurer des déplacements de la zone ciblée du coeur dans un référentiel lié au réseau de phase (RES US), les déplacements de la zone ciblée du coeur étant mesurés par le système de positionnement (SYS POS) configuré pour :
▪ mesurer les mouvements respiratoires du coeur dans un référentiel lié au réseau de phase (RES US) au moyen dudit système de positionnement (SYS POS), et en déduire un premier paramètre de compensation pour calculer une nouvelle position de la zone ciblée, ledit réseau de phase (RES US) étant configuré pour appliquer automatiquement un paramètre de phase à chaque signal pour défléchir le faisceau vers la nouvelle position de la zone ciblée (Z_{C}), et
▪ mesurer les mouvements de contraction du coeur dans un référentiel lié au réseau de phase (RES US) au moyen dudit système de positionnement (SYS POS) et en déduire un deuxième paramètre de compensation pour calculer une nouvelle position de la zone ciblée, ledit réseau de phase (RES US) étant configuré pour appliquer automatiquement un paramètre de phase à chaque signal pour défléchir le faisceau vers la nouvelle position de la zone ciblée (Z_{C}), et
▪ déduire un paramètre de compensation pour calculer une nouvelle position de la zone ciblée (ZC) et contrôler la déflexion du faisceau de signaux ultrasonores (FUS), ledit réseau de phase (RES US) étant configuré pour appliquer automatiquement un paramètre de phase à chaque signal pour défléchir le faisceau vers la nouvelle position de la zone ciblée (Z_{C}).

10. Système selon l'une quelconque des revendications précédentes **caractérisé en ce que** le système de positionnement (SYS POS) est configuré pour réaliser un asservissement dynamique de la désactivation et de l'activation des éléments du réseau de phase (RES US) en fonction du calcul de chaque paramètre de phase appliqué à chacun des signaux.

11. Système selon l'une quelconque des revendications précédentes **caractérisé en ce que** le système d'imagerie est configuré pour déterminer en outre une image d'une paroi transcostale projetée dans un plan image du réseau de phase (RES_US) en prenant en considération la position et l'orientation du réseau de phase-_et permettant de désactiver des éléments du réseau de phase en fonction de la position desdits éléments vis-à-vis de la position de l'image projetée de la paroi transcostale.

## Patentansprüche

1. System zur Ablation oder zur Kontrolle eines Herzbereichs durch Ultraschall, welches umfasst:
- ein Messmittel der elektrischen Herzaktivität für das Erfassen mindestens einer Frequenz eines Elektrokardiogramms (EKG);
- ein mit dem Messmittel der elektrischen Herzaktivität gekoppeltes Phasennetz für die anhand des Elektrokardiogramms des Herzens (EKG) erlangte frequenz synchrone Generierung eines auf mindestens einen anvisierten Bereich (Z_{c}) des Herzens fokussierten Ultraschallsignalstrahls (F_{US});
- ein mit dem Messmittel der elektrischen Herzaktivität und dem Phasennetz (RES_US) derart gekoppeltes Positionierungssystem, dass die Position mindestens eines anvisierten Bereichs von mindestens einem fokussierten Ultraschallsignalstrahl (F_{US}) auf die Position des mindestens einen anvisierten Bereich (Z_{c}) gesteuert wird,
- ein mit dem Messmittel der elektrischen Herzaktivität gekoppeltes MRT Bildgebungssystem, das konfiguriert ist, um eine aktive und dynamische Kontrolle einer Gewebetemperatur und Gewebeverformung in dem anvisierten Bereich (Zc) und/oder in den benachbarten Bereichen des anvisierten Bereichs (Zc) durchzuführen;
- eine Messvorrichtung für einen Kavitationsgrad im anvisierten Bereich (Zc);
- ein Rechner, der so konfiguriert ist, dass er während des Generierens des Ultraschallsignalstrahls (FUS) abhängig vom Kavitationsgrad und von der Gewebetemperatur und/oder Gewebeverformung im fokussierten Bereich die Merkmale dieses Ultraschallsignalstrahls (FUS) nach dessen Generierung dynamisch anpassen kann.

2. System nach Anspruch 1, in dem die Messvorrichtung eines Kavitationsgrades einen oder mehrere Ultraschallwellensensoren umfasst und in dem der Rechner eine Spektralanalyse ACQ_US der in oder in der Nähe des fokussierten Bereichs (ZF) reflektierten Ultraschallwellen vornimmt, um während der Generierung des Ultraschallsignalstrahls (FUS) dessen Merkmale in Abhängigkeit des Kavitationsgrades dynamisch anzupassen.

3. System nach einem der vorausgehenden Ansprüche, **dadurch gekennzeichnet, dass** die Generierung des Ultraschallsignalstrahls (FUS) synchron zu mindestens einer erfassten Frequenz des Elektrokardiogramms (EKG) des Herzens verläuft.

4. System nach einem der vorausgehenden Ansprüche, **dadurch gekennzeichnet, dass** das Phasennetz den Ultraschallsignalstrahl (FUS) vor der T-Welle des Elektrokardiogramms generiert.

5. System nach einem der vorausgehenden Ansprüche, **dadurch gekennzeichnet, dass** das Phasennetz einen Ultraschallsignalstrahl (FUS) mit einer durchgehenden Amplitude in einem Wertebereich von 1 - 100 MPa und einer Anwendungsdauer in einer Periode von über 1 ms generiert.

6. System nach einem der vorausgehenden Ansprüche, **dadurch gekennzeichnet, dass** das zur Messung einer Gewebetemperatur und Gewebeverformung in dem anvisierten Bereich (Zc) fähige Positionierungs- und Kontrollsystem das gleiche Bildgebungssystem verwenden, wobei es sich dabei um ein MRT-Bildgebungssystem handelt.

7. System nach einem der vorausgehenden Ansprüche, **dadurch gekennzeichnet, dass** der Rechner konfiguriert ist, um während der Generierung des Ultraschallsignalstrahls einen Indikator für eine Schädigung oder für eine Nekrose des anvisierten Bereichs (Zc) zu berechnen, wobei der Indikator durch Vergleich einer Messung eines Kavitationsgrads mit einem ersten Grenzwert und durch Vergleich einer Messung eines Verformungsgrads mit einem zweiten Grenzwert berechnet wird.

8. System nach einem der vorausgehenden Ansprüche, **dadurch gekennzeichnet, dass** die Signale des Ultraschallsignalstrahls (FUS) phasenkonfiguriert sind, um mindestens einen Impuls in einem fokussierten Bereich (ZF) zu generieren, und dadurch, dass ein Vergleich der Position des ermittelten fokussierten Bereichs (Z_{F}) durch das Bildgebungssystem mit der Position des durch das Positionierungssystem ermittelten anvisierten Bereichs (Z_{c}) mindestens einen Datensatz generiert, um Elemente des Phasennetzes derart zu kalibrieren, dass eine Position des fokussierten Bereichs mit der Position des anvisierten Bereichs übereinstimmt.

9. System nach einem der vorausgehenden Ansprüche, **dadurch gekennzeichnet, dass** die dynamische Steuerung der Position des fokussierten Bereichs (Z_{F}) mittels des Positionierungssystems (SYS_POS) in Bezug zur Position des anvisierten Bereichs (Z_{c}) ermöglicht, in einem phasennetzverbundenen (RES_US) Referenzsystem Bewegungen des anvisierten Bereichs im Herz zu messen, wobei die Bewegungen des anvisierten Bereichs im Herz durch das Positionierungssystem (SYS_POS) gemessen werden, welches konfiguriert ist, um:
- mittels des Positionierungssystems (SYS_POS) die Atembewegungen des Herzens in einem phasennetzverbundenen (RES_US) Referenzsystem zu messen und davon einen ersten Kompensationsparameter abzuleiten, um eine neue Position des anvisierten Bereichs zu berechnen, wobei das Phasennetz (RES_US) so konfiguriert ist, dass an jedem Signal automatisch ein Phasenparameter angewendet wird, um den Strahl in Richtung der neuen Position des anvisierten Bereichs (Z_{c}) zu lenken, und
- mittels des Positionierungssystems (SYS_POS) die Kontraktionsbewegungen des Herzens in einem phasennetzverbundenen (RES_US) Referenzsystem zu messen und davon einen zweiten Kompensationsparameter abzuleiten, um eine neue Position des anvisierten Bereichs zu berechnen, wobei das Phasennetz (RES_US) so konfiguriert ist, dass an jedem Signal automatisch ein Phasenparameter angewendet wird, um den Strahl in Richtung der neuen Position des anvisierten Bereichs (Z_{c}) zu lenken, und
- einen Kompensationsparameter abzuleiten, um eine neue Position des anvisierten Bereichs (ZC) zu berechnen und die Ablenkung des Ultraschallsignalstrahls (FUS) zu kontrollieren, wobei das Phasennetz (RES_US) so konfiguriert ist, dass an jedem Signal automatisch ein Phasenparameter angewendet wird, um den Strahl in Richtung der neuen Position des anvisierten Bereichs (Z_{c}) zu lenken.

10. System nach einem der vorausgehenden Ansprüche, **dadurch gekennzeichnet, dass** das Positionierungssystem (SYS_POS) konfiguriert ist, um in Abhängigkeit der Berechnung jedes bei jedem einzelnen Signal angewendeten Phasenparameters eine dynamische Steuerung der Deaktivierung und Aktivierung der Phasennetzelemente (RES_US) zu erzielen.

11. System nach einem der vorausgehenden Ansprüche, **dadurch gekennzeichnet, dass** das Bildgebungssystem konfiguriert ist, um zudem ein in einer Bildebene des Phasennetzes (RES_US) projiziertes Bild einer transkostalen Wand zu ermitteln und dabei die Position und die Ausrichtung des Phasennetzes zu berücksichtigen und zu ermöglichen, Elemente des Phasennetzes in Abhängigkeit der Position dieser Elemente gegenüber der Position des projizierten Bildes der transkostalen Wand zu deaktivieren.

## Claims

1. A system for ultrasonically ablating or checking a zone of the heart, comprising:
▪ a means for measuring the electrical activity of the heart for acquiring at least one frequency of an electrocardiogram (ECG);
▪ a phase grating coupled to the means for measuring the electrical activity of the heart for generating, in synchronisation with the acquired frequency of the electrocardiogram of the heart (ECG), a focused beam of ultrasound signals (Fᵤₛ) to at least one targeted zone (Z_{c}) of the heart;
▪ a positioning system coupled to the means for measuring the electrical activity of the heart and coupled to the phase grating (RES_US) so as to feedback control the position of at least one focused zone of at least one focused beam of ultrasound signals (Fᵤₛ) to the position of the at least one targeted zone (Z_{c}),
▪ an MRI imaging system coupled to the means for measuring the electrical activity of the heart and configured to carry out active dynamic check of a temperature and tissue deformation in the targeted zone (Z_{c}) and/or in the neighbouring zones of the targeted zone (Z_{c});
▪ a device for measuring a cavitation level in the targeted zone (Z_{c});
▪ a calculator configured to dynamically adjust, while generating the beam of ultrasound signals (Fᵤₛ), the characteristics of said beam of ultrasound signals (Fᵤₛ) as a function of the cavitation level and as a function of the temperature and/or tissue deformation in the focused zone in response to generating the beam of ultrasound signals (Fᵤₛ).

2. The system according to claim 1, wherein the device for measuring a cavitation level comprises one or more ultrasound wave sensors and wherein the calculator performs a spectral analysis ACQ_US of the ultrasound waves reflected in or in proximity to the focused zone (ZF) to dynamically adjust, while generating the beam of ultrasound signals (Fᵤₛ), the characteristics of said beam of ultrasound signals (Fᵤₛ) as a function of the cavitation level.

3. The system according to any of the previous claims, **characterised in that** generating the beam of ultrasound signals (Fᵤₛ) is synchronised to at least one acquired frequency of the electrocardiogram (ECG) of the heart.

4. The system according to any of the previous claims, **characterised in that** the phase grating generates the beam of ultrasound signals (Fᵤₛ) before the wave T of the electrocardiogram.

5. The system according to any of the previous claims, **characterised in that** the phase grating generates a beam of ultrasound signals (Fᵤₛ) of a continuous amplitude and included in a value range [1-100 MPa] and an application duration over a period of time longer than 1 ms.

6. The system according to any of the previous claims, **characterised in that** the positioning system and control system able to measure a temperature and tissue deformation in the targeted zone (Z_{c}) employs a same imaging system, said imaging system being an MRI imaging system.

7. The system according to any of the previous claims, **characterised in that** the calculator is configured to calculate, while generating the beam of ultrasound signals, an indicator either of a damage, or of a necrosis of the targeted zone (Z_{c}), the indicator being calculated by comparing a measurement of a cavitation level with a first threshold and by comparing a measurement of a deformation level with a second threshold.

8. The system according to any of the previous claims, **characterised in that** the signals of the beam of ultrasound signals (FUS) are configured in phase to generate at least one pulse in a focused zone (ZF), and **in that** comparing the position of the focused zone (Z_{F}) determined by the imaging system and the position of the targeted zone (Z_{c}) determined by the positioning system generates at least one piece of data to calibrate elements of the phase grating so as to match a position of the focused zone with the position of the targeted zone.

9. The system according to any of the previous claims, **characterised in that** the dynamic feedback control of the position of the focused zone (Z_{F}) to the position of the targeted zone (Z_{c}) by means of the positioning system (SYS_POS) enables movements of the targeted zone of the heart to be measured in a frame of reference related to the phase grating (RES_US), the movements of the targeted zone of the heart being measured by the positioning system (SYS_POS) configured to:
▪ measure respiratory movements of the heart in a frame of reference related to the phase grating (RES_US) by means of said positioning system (SYS_POS), and deduce therefrom a first compensation parameter to calculate a new position of the targeted zone, said phase grating (RES_US) being configured to automatically apply a phase parameter to each signal to deflect the beam towards the new position of the targeted zone (Z_{c}), and
▪ measure contraction movements of the heart in a frame of reference related to the phase grating (RES_US) by means of said positioning system (SYS_POS), and deduce therefrom a second compensation parameter to calculate a new position of the targeted zone, said phase grating (RES_US) being configured to automatically apply a phase parameter to each signal to deflect the beam towards the new position of the targeted zone (Z_{c}), and
▪ deduce a compensation parameter to calculate a new position of the targeted zone (ZC) and check deflection of the beam of ultrasound signals (FUS), said phase grating (RES_US) being configured to automatically apply a phase parameter to each signal to deflect the beam towards the new position of the targeted zone (Z_{c}).

10. The system according to any of the previous claims, **characterised in that** the positioning system (SYS_POS) is configured to carry out automatic feedback control of deactivation and activation of the elements of the phase grating (RES_US) as a function of the calculation of each phase parameter applied to each of the signals.

11. The system according to any of the previous claims, **characterised in that** the imaging system is configured to further determine an image of a transcostal wall projected in an image plane of the phase grating (RES_US) by taking the position and orientation of the phase grating into consideration and for deactivating the elements of the phase grating as a function of the position of said elements with respect of the position of the projected image of the transcostal wall.
